# EUROPÄISCHE PATENTSCHRIFT

(11) **EP 2 490 595 B1**
(45) Veröffentlichungstag und Bekanntmachung des Hinweises auf die Patenterteilung: **03.05.2017**
(21) Anmeldenummer: 10798250.6
(22) Anmeldetag: 20.10.2010
(51) Int. Cl.: A61B 10/02, A61B 10/04, G01N 33/543

(54) **BIOPSIE-INTRUMENT UMFASSEND EIN MAGNETISCHES ELEMENT**
BIOPSY INSTRUMENT COMPRISING A MAGNETIC ELEMENT
INSTRUMENT DE BIOPSIE COMPRENANT UN ÉLÉMENT MAGNÉTIQUE

(30) Priorität: 20.10.2009 DE 102009050361; 04.06.2010 DE 102010017236
(43) Veröffentlichungstag der Anmeldung: 29.08.2012
(73) Patentinhaber: OGENO GMBH, 14195 Berlin (DE)
(72) Erfinder: PISON, Ulrich, 14163 Berlin (DE); MEISSNER, Wolfgang, 12209 Berlin (DE); PIETSCHMANN, Silvia, 12209 Berlin (DE)
(74) Vertreter: Hertin und Partner Rechts- und Patentanwälte PartG mbB
(86) Internationale Anmeldenummer: PCT/DE2010/001239
(87) Internationale Veröffentlichungsnummer: WO 2011/047671

(56) Entgegenhaltungen:
- WO-A1-2006/131400
- WO-A1-2010/025719
- WO-A2-2008/110392
- US-B1- 7 309 316

## Beschreibung

Die vorliegende Erfindung bezieht sich auf ein Biopsie-Instrument zur Anreicherung von spezifischem Probenmaterial, wobei das Biopsie-Instrument ein magnetisches Element aufweist. Außerdem bezieht sich die Erfindung auf ein Verfahren zur Bindung von Mikroorganismen und die Verwendung des Biopsie-Instrumentes zur Diagnose, sowie auf ein Biopsie-Kit zu der Anreicherung von Zell- oder Molekülproben in verschiedenen Körperhohlsystemen wie beispielsweise einem Blutgefäß, Spinalkanal, Gallengang, ableitende Harnwege von Menschen oder Tieren.

Bakterielle Infektionen verursachen im klinischen Alltag hinsichtlich Diagnose und gezielter Therapie vielfältige Probleme. Die Deutsche Sepsis Gesellschaft e.V. hat auf ihren Internetseiten die klinische Bedeutung der Sepsis umfassend dargestellt. Danach hat sich die Vorstellung zur Pathophysiologie dieses Krankheitsbildes dahingegen gewandelt, dass nicht mehr die Krankheitserreger ausgelöste Infektion im Mittelpunkt steht, sondern die systemische Immunantwort des Patienten, die durch unterschiedliche Stimuli induziert sein kann. Diese neue Sichtweise ist insbesondere durch den klinischen Alltag geprägt, wo die mikrobiologische Diagnostik keine wesentlichen Entscheidungshilfen für die Therapie leistet. Der Identifikation und Sanierung eines Sepsisherdes und der raschen Antibiotikatherapie werden höchste Priorität eingeräumt.

Die Sterblichkeit an Sepsis ist seit Jahrzehnten mit 40% unverändert hoch. In den USA sterben jährlich ca. 263.000 Menschen an Sepsis und damit mehr als am akuten Herzinfarkt. Pro 100.000 Einwohner erkranken pro Jahr ca. 300 Menschen an schwerer Sepsis. Im Vergleich dazu sind es bei AIDS 17, bei Darmkrebs 50 und bei Brustkrebs 110 Neuerkrankungen pro 100.000 Einwohner. Die Sepsishäufigkeit hat sich in den vergangenen 20 Jahren in den USA verfünffacht. Patienten mit Sepsis müssen durchschnittlich 16 Tage auf Intensivstationen und 32 Tage im Krankenhaus behandelt werden. Für die meisten Europäischen Länder liegen die epidemiologischen Werte für die Sepsishäufigkeit niedriger als für die USA. So werden für Deutschland und Österreich 54-116 Fälle pro Jahr und 100.000 Einwohner angegeben. Die direkten anteiligen Kosten (Medikation, Routinelabor, Mikrobiologie, Einmalartikel, Unterkunft, Personal), die allein für die intensivmedizinische Behandlung von Patienten mit schwerer Sepsis anfallen, liegen bei ca. 1,77 Milliarden Euro. Damit werden ca. 30% des Budgets für Intensivmedizin in die Behandlung der schweren Sepsis investiert. Die indirekten Kosten, welche durch Produktivitätsverlust entstehen, werden auf weitere ca. 4,5 Milliarden Euro geschätzt, sodass von Gesamtkosten i. H. von ca. 6,3 Milliarden Euro auszugehen ist, welche durch die schwere Sepsis in Deutschland verursacht wird.

Die mikrobiologischen Standardmethoden zur Diagnostik bakterieller Erreger haben sich seit 1881, als Pasteur und Sternberg erstmals Pneumokokken aus Sputum nachwiesen und Christian Gram seine heute als Standard verwendete Färbemethode etablierte, nicht wesentlich geändert.

Der Erregemachweis bei Sepsisverdacht z.B. erfolgt aus Blutkulturen. Der Keimnachweis in diesen kritisch kranken Patienten gelingt häufig allerdings nicht und die Antibiotikatherapie wird regelhaft ohne Kenntnis des Resistenzmusters in der Klinik initiiert. Durch einen frühzeitigeren Erregemachweis in diesen Patienten könnte die Antibiotikatherapie gezielter durchgeführt werden, die Mortalität und Morbidität verbessert und die Therapiekosten gesenkt werden.

Die Identifikation und Separation von einzelnen Zellpopulationen aus dem Blut in vitro gelingt heutzutage mit funktionalisierten magnetischen Nanopartikeln. Hierbei spielt die magnetische Suszeptibilität der Partikel eine wichtige Rolle, da dieses Verhalten die magnetophoretische Eigenschaft der eingesetzten Partikel im Wesentlichen bestimmt. Die Dekoration von E.coli ließ sich in vitro erfolgreich mit magnetischen Nanopartikeln realisieren und mit bildgebende Verfahren lassen sich magnetotaktische Bakterien in vivo darstellen.

Die Entnahme von Material aus einem lebenden menschlichen oder tierischen Körper für mikrobiologische Diagnostikverfahren erfolgt üblicherweise durch die Gewinnung einer soliden Gewebeprobe durch eine Biopsie oder durch die Entnahme von Körperflüssigkeiten, z.B. in Form einer Gefäßpunktion zur Gewinnung einer Blutprobe oder durch Pleurapunktion zur Gewinnung eines Pleuraergusses oder durch Gelenkpunktion zur Gewinnung von Synovialflüssigkeit oder durch Spinalkanalpunktion zur Gewinnung von Liquor oder generell durch die Punktion eines Körperhohlsystemes zur Gewinnung von Material in flüssiger oder flüssigkeits-ähnlicher Form. Für die mikrobiologische Diagnostik werden ferner auch Abstriche von Körperregionen vorgenommen.

Der Stand der Technik beschreibt bisher Biopsie-Instrumente, die neben den herkömmlichen Stanz-, Schnitt- und Saugverfahren Antigen-Antikörper und Ligand-Rezeptor Interaktionen nutzen, um das Biopsie-Material zu gewinnen bzw. anzureichern.

In der modernen Medizin wird die Biopsie, d.h. das Entfernen von Material aus einem lebenden menschlichen oder tierischen Körper zwecks Untersuchung mittels verschiedener Methoden wie beispielsweise der Mikroskopie, routinemäßig durchgeführt. Typischerweise wird ein derartiges Verfahren für die Diagnose von malignen und prämalignen Zelltransformationen, welche auf die Anwesenheit von kanzerösen oder präkanzerösen Tumoren hinweisen, verwendet. Die als Kembiopsien bekannten Routine-Techniken, bei welchen ganze Gewebesegmente zur histologischen Untersuchung entnommen werden, machen von verschiedenen Arten von hohlen nadelförmigen Biopsie-Instrumenten Gebrauch. Diese Instrumente weisen Kanülen mit einer scharfen frontseitigen Kante oder ein gekerbtes Stilett auf, um beim Einführen in den ausgewählten Körperbezirk in das Gewebe zu schneiden. Das als Biopsieprobe zu entfernende Gewebestück wird in der zylindrischen Bohrung der Kanüle aufgenommen. Beim Herausziehen des Biopsie-Instrumentes aus dem Körper wird die Probe mittels mechanischer Mittel oder Saugmittel in der zylindrischen Bohrung bewahrt und wird beim Herausziehvorgang vom Hauptkörper des Gewebes getrennt. Die entnommenen Proben haben typischerweise eine generell längliche zylindrische Form oder eine longitudinal halbzylindrische Form. Die Qualität, z. B. die Breite, die Länge und der Anteil an zerquetschten Zellen von Biopsieproben zur zytologischen oder histologischen Untersuchung ist ein wichtiger Faktor, welcher das Untersuchungsresultat beeinflusst. Gewebebiopsieproben sollten die Struktur des lebenden Gewebes so genau wie möglich widerspiegeln. Dementsprechend sollten Spannungen auf die Proben beim Exzidieren und Abtrennen vom Gewebe sowie beim Entfernen aus der Kanüle vermieden werden.

Der Stand der Technik beschreibt bisher Biopsie Instrumente, die neben den herkömmlichen Stanz-, Schnitt- und Saugverfahren Antigen-Antikörper und Ligand-Rezeptor Interaktionen nutzen, um das Biopsie Material zu gewinnen.

Das US-Patent 5,282,476 beschreibt ein automatisiertes Gerät für die Biopsie, in welchem die Kanüle über einem Stilett angebracht ist, das die Kanüle zur Biopsiestelle lenkt. Nach dem Einführen wird die Kanüle über das Stilett hinaus getrieben und entnimmt dabei die Probe. Saugmittel werden zum Festhalten der Probe verwendet. Allerdings hat dieses Biopsie-Instrument wegen der Saugmittel einen ziemlich komplexen Aufbau und erfordert, beträchtliche Fähigkeiten bei der Handhabung. In den Händen eines ungeübten Anwenders kann das Instrument ungewisse Resultate ergeben. Ferner ist es für die Entnahme von Material aus soliden Körperstrukturen und nicht aus Körperhohlsystemen konzipiert.

Des Weiteren sind Instrumente oder Sensoren bekannt, die der Anreicherung von Proben dienen. Die WO/2006/131400 beschreibt die Nutzung einer funktionalisierten Oberfläche für die Anreicherung und Gewinnung von Molekülen oder seltenen Zellen, wie z.B. von fötalen Trophoblasten aus dem mütterlichen Blutkreislauf. Hierfür sind Rezeptorstrukturen auf der Oberfläche des Sensors verankert, die mit spezifischen Zellen oder Molekülen im Blutkreislauf reagieren und somit eine Anreicherung dieses Materiales *in situ* vor der Entnahme aus dem Körper gestatten sollen.

Weiterhin sind im Stand der Technik Vorrichtungen beschrieben (beispielsweise WO 01/23031 A1, EP 1 779 816 A2, US 2003/0135153, WO 98/22022 A1), mit deren Hilfe bestimmte Wirkstoffe oder Farbstoffe in Körperhöhlen einbringbar sind. Hierbei werden die Wirkstoffe in Aushöhlungen der Vorrichtungen eingebracht und beim Erreichen der Zielposition freigegeben. Nachteilig hierbei ist, dass die Proben sehr ungenau abgegeben werden und die Wirkstoffe in flüssiger Form in die Vorrichtungen einzubringen sind. Hierdurch sind die Einsatzmöglichkeiten der Vorrichtungen stark eingeschränkt.

WO 2008/110392 offenbart eine Biopsie-Vorrichtung für die in vivo-Anreicherung von Gewebe, Zellen oder von Analyten, wobei die Vorrichtung mit Gold oder Metallen, vorzugsweise aus der Gruppe 10 oder 11 des Periodensystems, beschichtet ist und ein Biopsie-Instrument nach der Präambel des Anspruchs 1. WO 2010/025719 offenbart ein Biopsie-Instrument zur Anreicherung von spezifischem Probenmaterial und die Verwendung des Biopsie-Instrumentes zur Diagnose. Das Instrument umfasst ein Biofunktionselement, welches zwischen dem distalen und dem proximalen Bereich eines Führungselementes angebracht ist und dessen Oberfläche Detektionsmoleküle aufweist. Magnetische Elemente sind in WO 2010/025719 nicht erwähnt.

Es sind außerdem magnetische Vorrichtungen im Stand der Technik beschrieben, die vollständig oder im Wesentlichen vollständig magnetisch sind. Die Vorrichtungen ermöglichen jedoch keine optimale Probenanreicherung oder -entnahme. Beispielsweise offenbart US 7309316 ein Verfahren zur magnetischen Nadelbiopsie, welches durch die Magnetisierung eines Biopsie-Gerätes superparamagnetische Nanopartikel isolieren kann.

Die im Stand der Technik beschriebenen Mittel zu der Entnahme oder Anreicherung von Proben sind nicht in allen Körperregionen einsetzbar. Insbesondere ist die endoskopische Verwendung beschriebener Instrumente/Sensoren nicht möglich oder nur schwer durchführbar. Andere wichtige Nachteile sind die Genauigkeit, mit welcher der für die Biopsie ausgewählte Gewebebezirk insbesondere Körperhohlsysteme mit dem Instrument/Sensor getroffen werden kann, die Einfachheit, mit welcher das Instrument/Sensor gehandhabt werden kann, die dem Patienten durch das Verfahren zugeführte Verletzung sowie die Kosten des Instrumentes oder des Sensors. Außerdem ist kein Biopsie-Instrument beschrieben, dass für den Einsatz bei Mensch und Tier konzipiert ist, um vor der Probenentnahme aus Körperhohlsystemen eine Anreicherung des Biopsiematerials risikoarm zu ermöglichen.

Bei Applikationen von Biopsie-Instrumenten, die über Antigen-Antikörper oder generell über Ligand-Rezeptor Interaktionen Biopsie-Material zu gewinnen suchen und dabei zu Anreicherungszwecken für eine definierte Zeit im Körper implantiert werden, hat sich herausgestellt, dass mehrere Faktoren diese Anreicherung unter Strömungsverhältnissen erschweren. Dazu zählt die Affinität des genutzten Liganden zu seinem korrespondierenden Rezeptor, die Viskosität des Strömungsmittels (z.B. Blut im Kreislaufsystem), die Strömungsverhältnisse (Geschwindigkeit, Strömungsform), die Größe des Zielobjektes und die räumliche Distanz, über die Anziehungskräfte eine Anreicherung von Analyten bewirken sollen.

Ausgehend von diesem Stand der Technik ist es die Aufgabe der vorliegenden Erfindung, ein Biopsie-Instrument bereitzustellen, das nicht die Nachteile oder Mängel des Stands der Technik aufweist und das die spezifische Anreicherung von Probenmaterial auch mittels endoskopischer Mittel und aus Körperhohlsystemen ermöglicht und über die Verweildauer im Körper nachgeschaltete diagnostische Verfahren erlaubt. Eine weitere Aufgabe der Erfindung ist die Entwicklung eines Biopsie-Instrumentes, das die Verletzungsgefahr für den Patienten minimiert, da besonders die Verweildauer des Biopsie-Instrumentes innerhalb des Körpers für das diagnostische Verfahren ein neues Kriterium bei der Probengewinnung ist.

Überraschenderweise wird diese Aufgabe durch die Merkmale der unabhängigen Ansprüche gelöst. Bevorzugte Ausführungsformen der Erfindung ergeben sich aus den Unteransprüchen.

Es war völlig überraschend dass ein Biopsie-Instrument zur Anreicherung von Probenmaterial bereitgestellt werden kann, was nicht die Nachteile und Mängel des Stands der Technik aufweist und folgende Komponenten umfasst:
i. ein Führungselement, umfassend einen feder-elastischen distalen und einen proximalen Bereich,
ii. ein magnetisches Element, welches zwischen dem distalen und dem proximalen Bereich des Führungselement angebracht ist, und
iii. ein mit dem proximalen Bereich des Führungselementes verbundenes Stabilisierungselement.

Überraschenderweise hat sich herausgestellt, dass die Nutzung eines magnetischen Elementes innerhalb eines Biopsie-Instrumentes dazu führt, dass Biopsiematerial auch unter erschwerten Flussbedingungen gewonnen werden kann. Bei im Stand der Technik offenbarten magnetischen Biopsiegeräten, die entweder vollständig magnetisch sind oder mehrere magnetische Elemente oder Bauteile umfassen, bestehen starke Wechselwirkungen zwischen den magnetischen Elementen, was die Probenentnahme erschwert. Außerdem hat sich herausgestellt, dass das hierdurch erzeugte Magnetfeld Einfluss auf Organe oder Gewebe haben kann. Dadurch, dass das erfindungsgemäße Biopsie-Instrument nur ein magnetisches Element umfasst, ist zum Einen das Magnetfeld so gering, dass keine Wechselwirkungen mit Geweben oder Organen entstehen und zum Anderen bestehen keine Wechselwirkungen innerhalb des Biopsie-Instrumentes.

Das Biopsie-Instrument wird zur Anreicherung von Probenmaterial genutzt. Hierbei kann es sich insbesondere um eine Probe aus der Gruppe umfassend Blut bzw. Blutplasma, Chylus bzw. Lymphe, Urin, Sperma, Vaginalsekret, Fruchtwasser, Speichel, Magensaft, Gallenflüssigkeit, Pankreassekret, Nasensekret, Bronchialsekret, Alveolarflüssigkeit, Liquor, Endolymphe, Kammerwasser, Tränenflüssigkeit, Synovialflüssigkeit, Pleuralflüssigkeit, Perikardflüssigkeit (Liquor pericardii), Peritonealflüssigkeit, Muttermilch, Schweiß, Menstruationsflüssigkeit oder Kombination hieraus oder anderen Proben aus dem Berech der Biopsie handeln.

Das Biopsie-Instrument besteht mindestens aus einem Führungselement, einem magnetischen Element und einem Stabilisierungselement. Das Führungselement weist einen distalen und einen proximalen Bereich auf, wobei der distale Bereich feder-elastisch ist. Im Sinne der Erfindung beschreiben sich die Begriffe "proximal" und "distal" insbesondere auf die Person, welche eine Biopsieprobe entnimmt. Dementsprechend ist das proximale Ende des Biopsie-Instrumentes insbesondere das rückwärtige Ende, das vom Patienten weg weist.

Der Begriff feder-elastisch beschreibt im Sinne der Erfindung insbesondere die Eigenschaft eines Materials, sich unter Belastung zu verformen und bei Entlastung wieder die im Wesentlichen ursprüngliche Gestalt anzunehmen. Durch diese Eigenschaft des distalen Bereichs des Führungselementes wird vorteilhafterweise sichergestellt, dass eine geringe bis keine Verletzungsgefahr für den Patienten besteht, da sich der feder-elastische Bereich bei Kontakt mit einer Oberfläche, wie z.B. einer Gefäßwand oder einem Organ, verformt und somit der Druck auf die Oberfläche reduziert wird.

Zwischen dem distalen und dem proximalen Bereich des Führungselementes ist ein magnetisches Element angebracht. Ein magnetisches Element bezeichnet im Sinne der Erfindung insbesondere eine Element, das vorteilhafterweise über ein Magnetfeld verfügt oder ein Magnetfeld erzeugen kann. Das magnetische Element kann aus einem magnetischen Material gefertigt sein oder eine magnetische Legierung aufweisen. Es kann jedoch auch bevorzugt sein, dass ein Magnetklebeband auf das magnetische Element aufgebracht wird. Es kann dementsprechend bevorzugt sein, dass das magnetische Element magnetische Werkstoffe, umfassend Metall, Kunststoff und/oder keramische Werkstoffe. Diese bezeichnen im Sinne der Erfindung insbesondere Werkstoffe, die beispielsweise unter Einwirkung eines äußeren Magnetfelds insbesondere auf Dauer magnetisiert werden. Die magnetischen Werkstoffe umfassen vorteilhafterweise ferromagnetische Materialien, bei denen die Magnetisierung durch ggf. sprunghafte Ausrichtung der Weißschen Bezirke unter dem Einfluß eines äußeren Magnetfeldes eintritt. Bevorzugte ferromagnetische Materialien umfassen Eisen oder Weicheisen, Siliciumeisen, Permalloy, Supermalloy, Cobalt, Nickel, Mn-Zn-Ferrite und Alnico V, Lanthanoide Gadolinium, Terbium, Dysprosium, Holmium und Erbium. Bevorzugt wird Neodym und/oder Samarium-Kobalt als magnetischer Werkstoff eingesetzt. Es kann jedoch auch bevorzugt sein, dass ein Elektromagnet als magnetisches Element verwendet wird. Der Elektromagnet kann vorteilhafterweise an- und abgeschaltet werden. Hierdurch ist es möglich, den Elektromagneten erst an dem Biopsieort zu aktivieren. Ein Elektromagnet umfasst insbesondere eine Spule, in der sich ein Kem aus einem ferromagnetischen Material befindet.

Vorteilhafterweise können auch Verbindungen als magnetisches Element verwendet werden, die kein ferromagnetisches Verhalten aufweisen, beispielsweise Chromdioxid, Manganarsenid, Europium(II)-oxid, suprafluide A-1 Phase von He-3 oder Heusler-Legierungen. Es können jedoch auch vorteilhafterweise Paramagnetika umfassend Mangan, Palladium und Chrom oder Ferrite mit der allgemeinen Zusammensetzung MIIFeIII₂O₄ oder MIIO Fe203, die permanente magnetische Dipole enthalten, als magnetisches Element verwendet werden. Bevorzugt werden Permanent-, Dauer- oder Hartmagnete als magnetisches Element eingesetzt. Vorteilhafte Dauermagnete umfassen AlNiCo-SmCo-, Nd₂Fe₁₄B-, Ni₈₀Fe₂₀, oder NiFeCo-Legierungen mit den Hauptbestandteilen Fe, Co, Ni, Al, Cu und Ti. Weitere bevorzugte magnetische Elemente umfassen PtCo-, FeCoVCr- und SECo-Legierungen. Da die Dauermagnete sehr hart und spröde sind, können hierdurch Biopsie-Instrumente mit überraschenden Vorteilen geformt werden. Es hat sich beispielsweise herausgestellt, dass hierdurch eine optimale Führung des Biopsie-Instrumentes durch Körperhöhlen möglich ist und es nicht anfällig für Strömungen ist.

Das magnetische Element kann bevorzugt auf seiner Oberfläche Detektionsmoleküle aufweisen, die mittels chemischer, elektrochemischer und/oder biologischer Verfahren auf dessen Oberfläche aufgebracht sind. Detektionsmoleküle sind dem Fachmann ebenfalls insbesondere als Fängermoleküle bekannt. Hierbei handelt es sich um Moleküle, die bevorzugt mit organischen und anorganischen Materialien interagieren. Als organische Materialien können z.B. Polymere verwendet werden. Etwa 90 % der organischen Materialien entstehen u. a. aus Kohlenstoff, Wasserstoff und Sauerstoff in wechselnden Mengenverhältnissen. Organische Verbindungen können auch Stickstoff, Schwefel, Phosphor und Halogene aber auch andere Elemente oder Kombinationen aus diesen enthalten. Des Weiteren können auch metallorganische Materialien, u. a. aufweisend eine Verbindung aus Metall und/oder Kohlenstoff verwendet werden. Als anorganische Materialien, d.h. nicht im Wesentlichen aus Kohlenstoff bestehen, können Materialien umfassend Glasarten, Keramik, Hartstoffe, nanostrukturierte und nanokristalline, Silicium oder Silikate eingesetzt werden. Somit kann bedingt durch die spezielle Ausgestaltung des magnetischen Elements eine Anreicherung von speziellen Zellen oder Molekülen im Körper von Mensch und Tier, d.h. *in situ,* erfolgen, was eine Abkehr vom technisch Üblichen darstellt und ein lang bestehendes Problem des Stands der Technik löst. Bedingt durch eine hohe Spezifität des magnetischen Elements wird eine schnelle Anreicherung von Probenmaterial erreicht, wodurch der Patient geschont und entlastet wird.

In dem proximalen Bereich des Führungselementes befindet sich das Stabilisierungselement, welches mit diesem Bereich fest verbunden oder frei verschiebbar gestaltet sein kann. Das Stabilisierungselement fixiert das magnetische Element während der Biopsie und verhindert so eine Dislokation des magnetischen Elements. Auch wird durch die erfindungsgemäße Ausführungsform des Biopsie-Instrumentes eine Entfernung des Instrumentes aus dem Körper des Patienten als Ganzes garantiert, d.h. durch die Anordnung der Komponenten wird eine Dislokation einzelner Komponenten verhindert, wodurch keine Komponente des Instrumentes im Körper des Patienten verbleibt.

Die erfindungsgemäße Lehre beschreibt demgemäß ein Biopsie-Instrument, welches in überraschend einfacher Weise ermöglicht, Zell- oder Molekülproben in verschiedenen Körperhohlsystemen von Mensch und Tier vor der Entnahme anzureichern, um, anders als bei den Vorrichtungen des Stands der Technik, eine optimale Konzentration für nachgeschaltete Diagnostikverfahren zu erreichen. Versuche haben gezeigt, dass Antikörper-Antigen-Bindungen nicht sehr stabil sind und somit gesammeltes Probenmaterial während der Entfernung oder Bewegung des Biopsie-Instrumentes verlorengehen. Die Interaktion zwischen Antigen-Antikörper wird zudem noch durch Strömungsverhältnisse erschwert, die an den Orten vorherrschen, wo das Biopsie-Instrument positioniert wird. Es war somit völlig überraschend, dass das erfindungsgemäße Biopsie-Instrument diese Nachteile nicht aufweist, da die Sammlung des biologischen Probenmaterials mittels einem magnetischen Elementes erreicht wird. Zu sammelnde spezifische Zellen oder Moleküle können vorteilhafterweise durch z. B. magnetische Nanopartikel markiert werden und durch das erfindungsgemäße Biopsie-Instrument, bzw. das magnetische Element gebunden werden. Die markierten Proben können ohne wesentliche Verluste aus einem Körper oder einem System isoliert werden.

Es war völlig überraschend, dass ein Biopsie-Instrument, bei dem lediglich ein einziges bestimmtes Element magnetisch ist, eine bisher unbekannte oder überlegene Wirkung gegenüber vollständig magnetischen Biopsie-Instrument aufweist, wobei das Instrument kein magnetisches Führungselement und kein magnetisches Stabilisierungselement aufweist. Bei Biopsie-Instrumenten, die vollständig oder im Wesentlichen vollständig magnetisch sind, bestehen starke magnetische Wechselwirkungen zwischen einzelnen Elementen, die negativ auf die Probenanreicherung wirken. Auch sind die im Stand der Technik beschriebenen magnetischen Biopsie-Instrumente sehr störanfällig und voluminös. Außerdem sind die Herstellung- und Wartungskosten solcher Instrumente sehr hoch. Es war völlig überraschend, dass das erfindungsgemäße Biopsie-Instrument diese Nachteile nicht aufweist. Das Biopsie-Instrument kann günstig hergestellt werden, wobei das magnetische Element einfach mit dem Führungselement verbindbar ist. Das Element kann nach einer Probenentnahme beispielsweise zur Probenanalyse weitergesendet werden und ein nicht benutztes magnetisches Element wird einfach und schnell auf dem Biopsie-Instrument angebracht. Vorteilhafterweise ist die Größe des magnetischen Elementes von der Größe des Biopsie-Instrumentes unabhängig, wodurch auch kleine und flexible Biopsie-Instrumente herstellbar sind.

Das magnetische Element des Biopsie-Instrumentes kann insbesondere aus ferromagnetischen Werkstoffen oder aus einem elektromagnetischen Detektor gefertigt sein. Mit beiden Techniken wird das gleiche Ziel erreicht, nämlich die effiziente und schnelle Anreicherung von spezifischem Probenmaterial, die Bindung von Mikroorganismen und der Einsatz als Diagnostikinstrument, sowie im Rahmen eines Biopsie-Kits zur Anreicherung von Zell-oder Molekülproben in verschiedenen Körperhohlsystemen.

Weitere Vorteile des erfindungsgemäßen Biopsie-Instrument sind die Genauigkeit mit welcher das Instrument in der für die Anreicherung von Probenmaterial ausgewählten Körperregion positioniert werden kann, die Spezifität und Sensitivität mit der sich Zellen und Moleküle in situ anreichem lassen und die Einfachheit, mit welcher das Biopsie-Instrument gehandhabt werden kann. Auch sind die Kosten für das Biopsie-Instrument gering. Eine schnelle und verlässliche Anreicherung von Proben zur Analyse, spielt in der Medizin eine wichtige Rolle und ist für die Diagnose von Krankheiten ein entscheidendes Kriterium.

Vorzugsweise sind die Komponenten des Biopsie-Instrumentes sequentiell von distal nach proximal angeordnet. Nach dem distalen feder-elastischen Führungselement ist das magnetische Element angeordnet, wobei sich in dem proximalen Bereich des Führungselementes das Stabilisierungselement befindet. Durch diese Anordnung wird sichergestellt, dass bei der Einfuhr des Biopsie-Instrumentes in den Körper der feder-elastische Bereich distal angeordnet ist und somit eine Verletzungsgefahr für den Patienten minimiert wird. Das magnetische Element wird vorteilhafterweise von dem proximal gelegenen Stabilisierungselement fixiert. Somit wird eine Dislokation der Komponenten während der Probenanreicherung verhindert und ein Verlust einer Komponente des Biopsie-Instrumentes verhindert. Außerdem wird durch die sequentielle Anordnung der Komponenten die Zuverlässigkeit erhöht und die Effizienz einer Biopsie verbessert. Einem kundigen Fachmann ist es möglich, Sonderformen des Biopsie-Instrumentes zu konstruieren, bei denen ein magnetisches Elemente mit unterschiedlichen Detektionsmolekülen zwischen distalem und proximalem Anteil des Führungselementes angeordnet ist oder die Funktionalisierung des distalen Anteiles des Führungselementes mit Detektionsmolekülen stattfindet unter Erhalt der feder-elastische Materialeigenschaften dieser Region.

Es ist weiterhin bevorzugt, dass der distale Bereich des Biopsie-Instrumentes, also der feder-elastische Anteil des Führungselementes, aus nicht magnetischen metallischen oder nicht-metallischen Materialien gefertigt ist. Dementsprechend kann es in einer bevorzugten Ausführungsform aus rostfreiem Stahl aber auch anderen Metallen gefertigt werden. Metalle bezeichnen chemische Elemente, die sich im Gegensatz zu den Nichtmetallen im Periodensystem links der diagonalen Trennungslinie beginnend mit dem Element Beryllium (2. Gruppe) bis hin zum Polonium (16. Gruppe) befinden, sowie deren Legierungen und intermetallische Verbindungen (umfassend Laves-Phasen, Heusler-Phasen, Zintl-Phasen, Hume-Rothery-Phasen, NiTi, Co5, Nb3Sn oder Ni3Al) mit charakteristischen metallischen Eigenschaften. Metalle umfassen Aluminium, Beryllium, Bismut, Blei, Chrom, Eisen, Gold, Indium, Kalium, Cobalt, Kupfer, Magnesium, Mangan, Molybdän, Natrium, Nickel, Osmium, Palladium, Platin, Rhodium, Ruthenium, Silber, Tantal, Titan, Vanadium, Wolfram, Zink, Zinn oder Zirconium. In einer vorteilhafter Ausführungsform kann ein innerer Draht von einem äußeren Draht zirkulär umgeben sein, so dass sich die feder-elastische Eigenschaft einstellt. Es ist zu betonen, dass im Sinne der Erfindung lediglich das magnetische Element aus einem magnetischen Material oder Werkstoff besteht.

In einer weiteren Ausführungsform kann das Biopsie-Instrument, insbesondere der distale Bereich des Führungselementes aus nichtmetallischen Materialien gefertigt sein, aus denen sich dünne zylindrische Strukturen formen lassen. Bevorzugte Nichtmetalle umfassen Bor, Kohlenstoff, Phosphor, Schwefel, Iod, oder Astat. Ein Zylinder ist im Sinne der Erfindung insbesondere eine Fläche, die von zwei parallelen, ebene Flächen (Grund- und Deckfläche) und einer Mantel- bzw. Zylinderfläche, die von parallelen Geraden gebildet wird, begrenzt ist. Es kann jedoch auch bevorzugt sein, dass der distale Bereich eckig ausgeformt ist.

Für nicht-metallische Materialien können in einer bevorzugten Ausführungsform Polymere verwendet werden. Polymere bezeichnen im Sinne der Erfindung insbesondere eine Substanz, die sich aus einem Kollektiv chemisch einheitlich aufgebauter, sich in der Regel aber hinsichtlich Polymerisationsgrad, Molmasse und Kettenlänge unterscheidender Makromoleküle (Polymermoleküle) zusammensetzt. Bei solchen polymereinheitlichen Stoffen sind alle Makromoleküle bevorzugt gleich aufgebaut und unterscheiden sich lediglich durch ihre Kettenlänge (Polymerisationsgrad). Man kann derartige Polymere als Polymerhomologe bezeichnen. Polymere können aus der Gruppe umfassend anorganische Polymere, metallorganische Polymere voll- oder teilaromatischen Polymeren, Homopolymere, Copolymere, Biopolymere, chemisch modifizierte Polymere und/oder synthetische Polymere ausgewählt werden und umfassen Polyethylen, Polypropylen, Polyvinylchlorid, Polystyrol, Polymethylmethacrylat, Polyamid, Polyester, Polycarbonat, Polyethylenterephthalat, Polyethylenglykol, Dendrimere, Silikone, Proteine, DNA, RNA, Kohlenhydrate oder Polyhydroxxyalkanoate. Bevorzugt wird Polytetrafluorethylen verwendet. Der aus diesen Materialien gefertigte distale Bereich des Biopsie-Instrumentes stellt einen technischen Fortschritt dar, da durch die bevorzugte Ausführungsform die Verletzungsgefahr reduziert wird. Das Material weist federelastische Eigenschaften auf und verhindert so eine Verletzung der Oberflächen mit denen das Biopsie-Instrument in Kontakt kommt.

Weiterhin vorteilhaft ist, dass das Stabilisierungselement des Biopsie-Instrument im Wesentlichen zylindrisch ausgebildet ist. Durch die vorteilhafte Ausführungsform kann das Stabilisierungselement auf den proximalen Bereich des Führungselementes einfach aufgebracht werden, indem es auf den proximalen Bereich aufgeschoben wird. Des Weiteren wird durch die vorteilhafte zylindrische Form eine leichte Einfuhr und Ausfuhr des Biospie-Instrumentes erreicht und somit die Effektivität gesteigert.

Vorteilhaft ist ebenfalls, dass das Stabilisierungselement über den proximalen Teil des Führungselementes schiebbar ist. Das Stabilisierungselement kann über den proximalen Bereich des Führungselementes aufgebracht werden, ohne die weiteren Komponenten, im Wesentlichen das magnetische Element, entfernen zu müssen. Das Stabilisierungselement fixiert das magnetische Element, was durch eine passgenaue Anfertigung der Komponente erreicht wird. Passgenau im Sinne der Erfindung bezeichnet insbesondere, dass mindestens zwei Komponenten derart gefertigt sind, dass sie kompatibel sind und exakt in die für sie vorgesehene Position passen. Die Einfachheit, mit der das Stabilisierungselement auf den proximalen Bereich des Führungselementes aufgebracht wird, erleichtert die Arbeit des Fachmanns. Des Weiteren kann das Stabilisierungselement nach erfolgreicher Biopsie einfach entfernt werden und die angereicherten Proben des magnetische Elements weiter verarbeitet oder an entsprechende Bearbeitungsstellen weitergeleitet werden. Die vorteilhafte Ausführungsform stellt somit einen glücklichen Griff dar, da aus einer Vielzahl von Möglichkeiten eine bestimmte gewählt wurde, deren Ergebnis nicht vorausgesagt werden konnte, daher handelt es sich um ein patentwürdigen glücklichen Griff.

Vorteilhafterweise ist weiterhin vorgesehen, dass der Außendurchmesser des Stabilisierungselementes bevorzugt gleich dem Außendurchmesser des distalen Bereichs des Führungselementes ist. Hierdurch wird eine ebene, d.h. eine plane Oberfläche des Biopsie-Instrumentes geformt, wodurch eine Verletzung des Patienten stark reduziert ist. Des Weiteren wird durch die vorteilhafte Ausführungsform eine leichte Einfuhr des Biopsie-Instrumentes in Körperregionen garantiert und zusätzlich sichergestellt, dass es als Ganzes wieder entfernt wird. Im Ganzen bedeutet, dass durch die Anordnung der Komponenten eine Dislokation einzelner Komponenten verhindert wird, wodurch keine Komponente des Instrumentes im Körper des Patienten verbleibt.

Eine weitere vorteilhafte Ausführungsform umfasst ein Biopsie-Instrument wobei das Stabilisierungselement reversibel mit dem proximalen Bereich des Führungselementes verbunden ist. Vorzugsweise lässt sich das Stabilisierungselement gemäß dieser Ausführungsform, nach der Biopsie von dem proximalen Bereich des Führungselementes wieder entfernen, um das magnetische Element freizugeben. Das magnetische Element kann zur Durchführung von nachgeschalteten Diagnose-Verfahren in die entsprechenden Bearbeitungsstellen versendet werden oder es kann eine Analyse der angereicherten Proben vor Ort vorgenommen werden. Die vorteilhafte Ausführungsform stellt einen technischen Fortschritt dar, da der Fachmann das Biopsie-Instrument einfach zusammenbauen kann und es nach beendeter Probenanreicherung wieder in die einzelnen Komponenten zerlegen kann. Das Stabilisierungselement kann so mehrmals, nach entsprechender Desinfektion, genutzt werden, wodurch die Kosten reduziert werden.

Weiterhin ist vorteilhaft, wenn das Stabilisierungselement vorzugsweise aus Kunststoff gefertigt ist. Kunststoffe bezeichnen Materialien, deren wesentliche Bestandteile aus solchen makromolekularen organischen Verbindungen bestehen, die synthetisch oder durch Abwandeln von Naturprodukten entstehen. Sie sind in vielen Fällen unter bestimmten Bedingungen (Wärme u. Druck) schmelz- und formbar. Zu den Kunststoffen gehören auch die Kautschuke und die Chemiefasem. Auch die synthetischen Lackrohstoffe und die Klebstoffe können zu den Kunststoffen gezählt werden. Für die vorteilhafte Ausführungsform können Kunststoffe aus der Gruppe abgewandelte Naturstoffe, synthetische Kunststoffe (Polykondensate, Polymerisate, Polyaddukte), Duroplaste, und/oder ungesättigte Polyesterharze, umfassend Cellulosenitrat, Celluloseacetat, Cellulosemischester, Celluloseether, Polyamid, Polycarbonat, Polyester, Polyphenylenoxid, Polysulfon, Polyvinylacetal, Polyethylen, Polypropylen, Poly-1-buten, Poly-4-methyl-1-penten, lonomere, Polyvinylchlorid, Polyvinylidenchlorid, Polymethyl-methacrylat, Polyacrylnitril, Polystyrol, Polyacetal, Fluor-Kunststoffe, Polyvinylalkohol, Polyvinylacetat, Poly-p-xylylen, lineare Polyurethane, chlorierte Polyether, Casein-Kunststoffe, Phenol-Harz, Harnstoff-Harz, Thioharnstoff-Harz, Melamin-Harz, Epoxidharz, vernetzte Polyurethane, Alkydharz, Allylharz, Silicon, Polyimid, und/oder Polybenzimidazol verwendet werden. Durch die Verwendung von Kunststoff bei der Ausgestaltung des Stabilisierungselementes sind Biopsie-Instrumente mit gummi-elastischen Eigenschaften herstellbar, die sich an unterschiedlichen Biopsie-Orten im Körper platzieren lassen. Durch die vorteilhafte Ausführungsform kann somit eine erhöhte Zuverlässigkeit und Flexibilität bei der Durchführung einer Biopsie erreicht werden. Auch ist die Verletzungsgefahr des Patienten drastisch reduziert, da die gummi-elastische Ausführungsform keine Oberflächen verletzen. Des Weiteren können durch die Verwendung von Kunststoffen für die Ausgestaltung Kosten reduziert werden.

Vorteilhafterweise ist vorgesehen, dass das Stabilisierungselement und der proximale Bereich des Führungselementes mechanisch verbunden, verklebt und/oder verschweißt sind. Hierdurch wird eine stabile Verbindung des proximalen Bereichs des Führungselementes und des Stabilisierungselementes erreicht, wodurch die Handhabung des Biopsie-Instrumentes für den Fachmann erleichtert wird. Durch eine mechanische Verbindung kann die Verbindung reversibel gestaltet werden, wobei hierfür dem Fachmann bekannte Befestigungsmittel verwendet werden. Weiterhin sind dem Fachmann Verklebungen oder Verschweißungen bekannt, die eine sichere und permanente Verbindung des proximalen Bereichs und des Stabilisierungselementes sicherstellen. Durch die stabile Verbindung wird garantiert, dass das Biopsie-Instrument als Ganzes in einen Patienten eingeführt und auch nach Probenanreicherung wieder ausgeführt werden kann, wodurch die Zuverlässigkeit verbessert und der Biopsie-Prozess an sich vereinfacht wird.

Des Weiteren vorteilhaft ist, dass das Führungselement und das Stabilisierungselement in einem Herstellungsverfahren gefertigt sind, das heißt es können für eine Anwendung Materialien für beide Komponenten verwendet werden, die auf die entsprechenden Körperregionen in Größe und Form abgestimmt sind. Eine vorteilhafte Ausführungsform ist Kunststoff, aus der Gruppe abgewandelte Naturstoffe, synthetische Kunststoffe (Polykondensate, Polymerisate, Polyaddukte), Duroplaste, und/oder ungesättigte Polyesterharze, umfassend Cellulosenitrat, Celluloseacetat, Cellulosemischester, Celluloseether, Polyamid, Polycarbonat, Polyester, Polyphenylenoxid, Polysulfon, Polyvinylacetal, Polyethylen, Polypropylen, Poly-1-buten, Poly-4-methyl-1-penten, lonomere, Polyvinylchlorid, Polyvinylidenchlorid, Polymethyl-methacrylat, Polyacrylnitril, Polystyrol, Polyacetal, Fluor-Kunststoffe, Polyvinylalkohol, Polyvinylacetat, Poly-p-xylylen, lineare Polyurethane, chlorierte Polyether, Casein-Kunststoffe, Phenol-Harz, Harnstoff-Harz, Thioharnstoff-Harz, Melamin-Harz, Epoxidharz, vernetzte Polyurethane, Alkydharz, Allylharz, Silicon, Polyimid, und/oder Polybenzimidazol. Hierdurch kann erreicht werden, dass die Verletzungsgefahr für den Patienten reduziert wird, da eine ebene Oberfläche des Instrumentes entsteht. Auch wird für den Fachmann die Handhabung des Biopsie-Instrumentes vereinfacht und die Herstellungskosten für das Biopsie-Instrument können gering gehalten werden.

In einer vorteilhaften Ausführungsform ist vorgesehen, dass das magnetische Element eine im Wesentlichen zylindrische Form aufweist, wobei auch andere geometrische Formen verwendbar sind, umfassend Polygon, Dreieck, Viereck, Fünfeck, Sechseck, Siebeneck, Achteck, Neuneck, Zehneck, Ellipse, Kreis, Hyperbel, Parabel, Superellipse, Kugeldreieck, Zykloide, Rosette, Helix, Kugel, Ellipsoid, Rotationsellipsoid, Paraboloid, Rotationsparaboloid, Hyperboloid, Oloid oder Polyeder bzw. Kombinationen dieser oder andere Formen. Durch die Ausführungsform kann das magnetische Element leicht von proximal auf das Führungselement aufgebracht werden, wobei es nach dem Aufbringen räumlich von dem feder-elastischen distalen Bereich und dem proximalen Stabilisierungselement fixiert wird. Hierdurch ist eine Dislokation des magnetische Elements ausgeschlossen. Des Weiteren wird durch die bevorzugte im Wesentlichen zylindrische Form sichergestellt, dass das Biopsie-Instrument eine ebene Oberfläche aufweist und eine leichte Einfuhr und Ausfuhr des Biopsie-Instrumentes garantiert werden kann. Somit kann durch die vorteilhafte Ausführungsform die Qualität der Probenanreicherung erhöht werden wobei die Zeit der Probenanreicherung reduziert wird.

Es hat sich überraschenderweise herausgestellt, dass die Größe des magnetische Elements reduziert werden kann, wenn das Element eine raue Oberfläche aufweist. Durch die Rauheit kann die Oberfläche des Elementes vergrößert und die Dimensionen verkleinert werden. Die Dimension bezeichnet insbesondere Längen- und/oder Breitenabmessungen. Dies führt überraschenderweise zu einer effizienteren Probenanreicherung, was vorteilhaft gegenüber dem Stand der Technik ist. Die Verwendung eines magnetischen Elementes in einem Biopsie-Instrument stellt somit eine Abkehr von dem Üblichen dar, da im Stand der Technik lediglich Instrumente offenbart sind, die vollständig oder im Wesentlichen vollständig magnetisch sind. Durch die Verwendung eines kleinen magnetischen Elementes können jedoch zahlreiche Vorteile gegenüber dem Stand der Technik erreicht werden, wie beispielsweise geringere unspezifische Interaktionen, weniger störanfällig oder keine Wechselwirkungen innerhalb des Biopsie-Instrumentes.

Vorteilhafterweise hat das magnetische Element einen Außendurchmesser, der größer, gleich oder kleiner, vorzugsweise 0,01 bis 0,1 mm kleiner als der Außendurchmesser des distalen Bereichs des Führungselementes ist. Durch die vorteilhafte Ausführungsform wird erreicht, dass das magnetische Element von proximal auf das Führungselement aufgebracht werden kann, jedoch von dem distalen Bereich räumlich begrenzt wird. Der Außendurchmesser des magnetische Elements ist 0,0001 bis 5 mm, bevorzugt 0,001 bis 1 mm, besonders bevorzugt 0,01 bis 0,1 mm kleiner als der Außendurchmesser des distalen Bereichs des Führungselementes. Durch den bevorzugten Außendurchmesser des magnetische Elements kann eine nahezu ebene Oberfläche des Biopsie-Instrumentes erzeugt werden, wodurch überraschenderweise eine optimale Funktionalität des Instrumentes garantiert wird, da bei der Einfuhr und Ausfuhr des Biopsie-Instrumentes das Instrument nicht mit biologischen Oberflächen interagiert und so die Positionierung des Biopsie-Instrumentes nicht beeinträchtigt wird. Außerdem hat sich herausgestellt, dass durch den bevorzugten Außendurchmesser einer besonders effiziente Probenanreicherung erreicht werden kann und das Instrument schneller und einfacher zu sterilisieren bzw. zu reinigen ist. Der Außendurchmesser des Biopsie-Instrumentes und demgemäß der Außendurchmesser des magnetische Elements orientiert sich an der anatomischen Struktur, in der das Biopsie-Instrument eingesetzt werden soll. Die Länge ist abhängig von der Punktionsstelle und der Positionierung für die Probensammlung *in situ.* Die Dimensionen des Biopsie-Instrument können einfach und schnell an die Anforderungen der unterschiedlichen Biopsie-Orte angepasst werden, woraus ein Ersparnis an Zeit und Kosten resultiert.

Weiterhin ist vorteilhafterweise vorgesehen, dass das Führungselement zwischen dem distalen und proximalen Bereich eine Aufnahmevorrichtung aufweist, welche der Aufnahme des magnetische Elements dient. Bei der Aufnahmevorrichtung kann es sich um Aussparungen verschiedener geometrischer Formen handeln, umfassend, Polygon, Dreieck, Viereck, Fünfeck, Sechseck, Siebeneck, Achteck, Neuneck, Zehneck, Ellipse, Kreis, Hyperbel, Parabel, Superellipse, Kugeldreieck, Zykloide, Rosette, Helix, Kugel, Ellipsoid, Rotationsellipsoid, Paraboloid, Rotationsparaboloid, Hyperboloid, Oloid oder Polyeder oder Kombination dieser oder andere Formen. Eine vorteilhafte Ausführungsform umfasst zylindrisch, halbzylindrisch, spangenförmig oder folienförmig Aussparungen. Hierbei handelt es sich um Beispiele, die keine Limitierung der Aufnahmevorrichtung darstellen sollen. Folienförmig bezeichnet im Sinne der Erfindung insbesondere dass die Aufnahmevorrichtung oder das magnetische Element derart geformt sind, das sie eine sehr geringe Dicke und große Fläche aufweisen. Das magnetische Element wird bevorzugt entsprechend der Form der Aufnahmevorrichtung geformt. Auch diese Ausführungsalternativen stellen sicher, dass durch die vorteilhafte Anordnung einer distal gelegenen Führungselementes gefolgt von einem magnetischen Element sich ein Stabilisierungselement anschließt. So wird erreicht, dass bei minimaler Verletzungsgefahr für den Patienten die magnetische Funktion des Instrumentes gewährleistet bleibt und sich das Biopsie-Instrument nach Sammlung von Probenmaterial *in situ* in Gänze von außen aus dem Körper wieder entfernen lässt. Des Weiteren kann durch die vorteilhafte Ausführungsform das Biopsie-Instrument an unterschiedliche Anwendungen angepasst werden, wobei auch durch die Form der Aufnahmevorrichtung bzw. des magnetische Elements die zu sammelnde Probenmenge variiert und an den Bedarf des Anwenders angepasst werden kann. Es war völlig überraschend, dass die vorteilhafte Ausführungsform optimal an den Biopsieorten platzierbar ist und die Probenentnahme nicht durch ggf. auftretende Strömungseffekte beeinflusst wird, wodurch die Proben ohne Verlust entnommen werden können.

Vorteilhafterweise umfasst das magnetische Element Metall, Kunststoff, und/oder keramischen Werkstoffe. Metallen umfassen, wie bereits oben aufgeführt, Aluminium, Beryllium, Bismut, Blei, Chrom, Eisen, Gold, Indium, Kalium, Cobalt, Kupfer, Magnesium, Mangan, Molybdän, Natrium, Nickel, Osmium, Palladium, Platin, Rhodium, Ruthenium, Silber, Tantal, Titan, Vanadium, Wolfram, Zink, Zinn oder Zirconium. Keramische Werkstoffe bezeichnen eine Sammelbezeichnung für aus anorganischen und überwiegend nichtmetallischen Verbindungen oder Elementen aufgebaute und zu mehr als 30 Volumen-% kristallisierte Materialien. Keramische Werstoffen umfassen, Tonerde bzw. Kaolin, Quarz, Feldspat, Kalk, Silimanit, Magnesit, Terrakotta, Majolika, Fayence, Raku, Paperclay oder Oxidkeramiken. Für die vorteilhafte Ausführungsform können des Weiteren Kunststoffe, wie bereits oben bei den nicht magnetischen Kunststoffen erwähnt, verwendet werden. Die vorteilhafte Ausführungsform stellt sicher, dass eine effektive und schnelle Probenanreicherung durch das magnetische Element erreicht wird und das gesammelte Material an das magnetische Element gebunden wird und während der Entfernung des Biopsie-Instrumentes an diesem verweilt. Außerdem wird durch die Wahl des Werkstoffes, die Verletzungsgefahr für den Patienten reduziert. Eine Vergrößerung der Oberfläche der Werkstoffe ist zusätzlich möglich, um die Effektivität der Probenanreicherung zusätzlich zu steigern. Es hat sich herausgestellt, dass durch das magnetische Element eine effektive Probenanreicherung in beispielsweise Gefäßen möglich ist, wo eine hohe Strömung herrscht. Dies war völlig überraschend und stellt einen erheblichen Vorteil gegenüber dem Stand der Technik dar. Durch die bevorzugten magnetischen Eigenschaften des magnetischen Elements und die fehlenden störenden Wechselwirkungen innerhalb eines Biopsiegerätes des Stands der Technik, können auch Proben angereichert werden, die sich nicht in dem näheren Umfeld des Instrumentes befinden, da die magnetischen Kräfte so über eine längere Distanz wirken als Antigen-Antikörper-Bindungskräfte. Überraschenderweise bewirken die durch das magnetische Element erzeugten magnetischen Kräfte jedoch keine negative Beeinflussung von Geweben oder Organen, da diese schwächer als die der im Stand der Technik beschriebenen magnetischen Biopsiegeräte sind.

In einer weiteren vorteilhaften Ausführungsform ist vorgesehen, dass die Detektionsmoleküle bevorzugt aus der Gruppe umfassend Antikörper, Antikörperfragmente, synthetische Protein-Scaffolds, Peptide, Nukleinsäuren, Rezeptoren und/oder anorganischen Materialien ausgewählt sind. Das Aufbringen von Detektionsmolekülen auf dem magnetische Element erreicht eine spezifische Anreicherung von Proben *in situ* vor der Entnahme der Probe. Die Detektionsmoleküle können mit Zellen und/oder Molekülen im Körper von Mensch oder Tier reagieren und binden die Zellen und/oder Moleküle. Hierfür können unterschiedliche Detektionsmoleküle Verwendung finden und auch miteinander kombiniert werden. Durch die spezifische Positionierung des Biopsie-Instrumentes in einer gewünschten Körperregion und durch die Spezifität und Sensitivität des Instrumentes, kann erreicht werden, dass auch geringe Konzentrationen an Molekülen und/oder Zellen durch die Detektionsmoleküle detektiert werden. Dies stellt einen technischen Fortschritt dar. Des Weiteren ist durch eine Variation der Detektionsmoleküle ein breites Anwendungsfeld der vorteilhaften Ausführungsform denkbar. Es kann ebenfalls vorteilhaft sein, unterschiedliche Detektionsmoleküle auf dem magnetischen Element aufzubringen. So können beispielsweise unterschiedliche Detektionsmoleküle, die beispielsweise spezifisch für einen einzigen, oder für jeweils einen bestimmten Zelltyp sind, aufgebracht werden. Hierdurch wird sichergestellt, dass bevorzugt eine spezifische Probe angereichert wird. Die Moleküle oder Zellen werden schnell und effizient gesammelt und können anschließend vorteilhafterweise in einem Labor quantifiziert und analysiert werden. Besonders eine schnelle und zuverlässige Probenanreicherung spielt in der Medizin oder genauer der Diagnostik eine entscheidende Rolle für die Diagnose von Krankheiten.

Es ist bevorzugt, Liganden mit magnetischen Nanopartikeln zu verbinden. Hierfür können beispielsweise Eisenoxidpartikel mit unterschiedlicher Hülle verwendet werden. Die Partikel, die im Sinne der Erfindung auch als Nanoteilchen bezeichnet werden können, können hinsichtlich Größe und Ladung, sowie hinsichtlich ihres magnetischen Relaxationsverhaltens und auch hinsichtlich ihrer magnetischen Suszeptibilität angepasst werden und sind kolloidal stabil. Die mit den magnetischen Nanopartikeln verbundenen Liganden binden vorzugsweise an definierte Oberflächenmoleküle von Mikroorganismen, umfassend Bakterien, Viren oder Pilze. Die Mikroorganismen können durch die Dekorierung mit den magnetischen Nanopartikeln schnell und sicher von dem magnetischen Element des Biopsie-Instrumentes angereichert werden. Es kann weiterhin bevorzugt sein, magnetische Nanopartikel mit einem Hüllmaterial, umfassend Kohlenhydrate, Proteine, Nukleinsäuren und/oder Polymeren zu versehen, was von Mikroorganismen bevorzugt aufgenommen wird. Hierdurch ist ebenfalls eine magnetische Dekorierung der Mikroorganismen möglich, wobei insbesondere magnetisierte Mikroorganismen entstehen. Es war völlig überraschend, dass durch die magnetische Dekorierung der Mikroorganismen ein magnetisches Moment bereitgestellt werden kann, was eine ungestörte Attraktion der Mikroorganismen im Blutkreislauf gewährleistet. Hierdurch ist es überraschenderweise möglich, Infektionskrankheiten, die mit Mikroorganismen assoziiert sind, schnell und einfach zu diagnostizieren und/oder zu therapieren. Die magnetische Anreicherung erfolgt vorteilhafterweise ohne oder in Verbindung mit der Anreicherung durch die Detektionsmoleküle, die ebenfalls an Mikroorganismen binden können. Hierdurch kann eine zweifache Bindung der Mikroorganismen erreicht werden, was die Effizienz der Probenanreicherung erheblich verbessert. Außerdem ist es so möglich, Proben auch unter nicht-tubulären Strömungsverhältnissen anzureichern. Die Bindungskräfte von gängigen Ligand-Rezeptor-Interaktionen reichen häufig nicht aus, um Zielobjekte von der Größe einer Zelle unter physiologischen Strömungsverhältnissen zu isolieren. Die Integration eines zusätzlichen Bindungsvektors in Form des magnetischen Elementes ermöglicht es allerdings, dass sich auch größere Objekte unter physiologischen Strömungsverhältnissen isolieren lassen, ohne das magnetische Wechselwirkungen innerhalb des Biopsie-Instrumentes oder mit Geweben oder Organen entstehen. Ein weiterer Vorteil der Nutzung der magnetischen Anziehungskraft als Funktionsprinzip bei der Isolierung größerer Zielobjekte aus dem Blutkreislauf beruht darauf, dass diese Kräfte gewöhnlich über eine wesentlich größere Distanz wirken als Ligand-Rezeptor Interaktionen. Es war jedoch völlig überraschend, dass das magnetische Element gezielt magnetisierte Zielobjekte anzieht, wobei die im Stand der Technik beschriebenen magnetischen Biopsiegeräte ein ungenaues und großflächiges Magnetfeld aufweisen, was mit Wechselwirkungen innerhalb des Gerätes und mit Geweben und Organen einhergeht.

Weiterhin ist vorteilhafterweise vorgesehen, dass das Biopsie-Instrument zusätzlich eine Abdeckvorrichtung aufweist. Die Abdeckvorrichtung hat vorzugsweise eine hülsenförmige Struktur, die das magnetische Element abdeckt. Andere geometrische Formen, umfassend Polygon, Dreieck, Viereck, Fünfeck, Sechseck, Siebeneck, Achteck, Neuneck, Zehneck, Ellipse, Kreis, Hyperbel, Parabel, Superellipse, Kugeldreieck, Zykloide, Rosette, Helix, Kugel, Ellipsoid, Rotationsellipsoid, Paraboloid, Rotationsparaboloid, Hyperboloid, Oloid oder Polyeder sind ebenfalls verwendbar. Dadurch lässt sich das magnetische Element und das dort nach der Anreicherung *in situ* lokalisierte Probenmaterial vor der Entnahme aus dem Körper vor Abscherung oder Deformation schützen und eine Kontamination außerhalb des Körpers vermeiden. Eine vorteilhafte Ausführungsform ist dass die Abdeckvorrichtung eine Schiebevorrichtung darstellt, die von dem proximalen Bereich über das magnetische Element geschoben wird. Jedoch sind auch andere Vorrichtungen, umfassend Klappvorrichtungen denkbar, die der Abdeckung des magnetischen Elements dienen. Durch die vorteilhafte Ausführungsform wird die angereicherte Probe geschützt, wodurch Messfehler reduziert werden und die Qualität der Anreicherung verbessert wird.

Vorteilhaft ist, dass die Abdeckvorrichtung vorzugsweise das magnetische Element abdeckt, wobei die Abdeckvorrichtung insbesondere Homopolymere, Copolymere, Biopolymere, chemisch modifizierte Polymere und/oder synthetische Polymere umfasst. Als Materialien für die Abdeckvorrichtung eignen sich Polymermaterialien, umfassend Polyethylen, Polypropylen, Polyvinylchlorid, Polystyrol, Polymethylmethacrylat, Polyamid, Polyester, Polycarbonat, Polyethylenterephthalat, Polyethylenglykol, Dendrimere, Silikone, Proteine, DNA, RNA, Kohlenhydrate oder Polyhydroxxyalkanoate; vorzugsweise Polytetrafluorethylen. Insbesondere wenn die angrenzenden Komponenten aus dem gleichen Material gefertigt sind, wird dadurch eine gute Verschiebbarkeit der Einzelkomponenten erreicht. Durch die vorteilhafte Ausführungsform kann die Abdeckvorrichtung leicht über das magnetische Element geschoben werden oder dieses abdecken. Durch die vorteilhafte Ausführungsform werden Fehler bei der Biopsie-Entnahme reduziert und so die Effizienz verbessert und der Arbeitsaufwand reduziert.

Eine weitere vorteilhafte Ausführungsform ist, dass das Biopsie-Instrument eine longitudinale Bohrung aufweist, zur Aufnahme bevorzugt eines Mandrains und/oder anderer Funktionselemente. Die Bohrung kann durch dem Fachmann bekannte Verfahren in das Biopsie-Instrument eingebracht werden und die Breite der Bohrung richtet sich nach der Anwendung und kann dementsprechend variieren. In den longitudinalen dünnen Bohrkanal kann z.B. ein Mandrain (ein Führungsdraht) eingebracht werden und bis in das distale Ende des Biopsie-Instrumentes vorgeschoben werden. So kann die Steifigkeit des Instrumentes für Platzierungsmanöver im Körper optimiert werden. Auch können in eine solche Bohrung weitere Funktionalisierungen z.B. elektronischer Art eingebracht werden, um die Nutzbarkeit des Biopsie-Instrumentes zu erhöhen. Die longitudinale Bohrung verbessert die Arbeitssicherheit und stellt eine Vereinfachung dar.

Desweiteren sind Gestaltungsformen des Biopsie-Instrumentes möglich, die folgende Spezialfälle beinhalten aber nicht darauf beschränkt sind und durch den kundigen Fachmann leicht ausgeführt werden können: die Anordnung von zwei oder mehr magnetischen Teilelementen innerhalb des magnetischen Elementen bevorzugt mit unterschiedlichen Detektionsmolekülen zwischen distalem und proximalem Anteil des Führungselementes; und/oder die Funktionalisierung des distalen Anteiles des Führungselementes mit Detektionsmolekülen unter Erhalt der feder-elastischen Materialeigenschaft dieser Region. Es kann dementsprechend vorteilhaft sein, wenn das magnetische Element ein oder mehrere separate oder miteinander verbundene magnetische Teilelemente umfasst.

Es ist weiterhin auf Basis des erfindungsgemäßen Biopsie-Instrumentes möglich, ein Verfahren zur Bindung von Mikroorganismen bereitzustellen, das ein erfindungsgemäßes Biopsie-Instrument umfasst, wobei das Verfahren folgende Schritte umfasst:
i. Kopplung von Liganden an magnetische Nanopartikel;
ii. Bindung der Liganden an Mikroorganismen;
iii. Bindung der magnetischen Nanopartikel an mindestens ein magnetisches Element des Biopsie-Instruments.

Liganden werden bevorzugt mit magnetischen Nanopartikeln gekoppelt, wobei die Liganden bevorzugt spezifisch für Mikroorganismen sind. Die Liganden binden bevorzugt an die Oberfläche von Mikroorganismen, wodurch diese durch die magnetischen Nanopartikel magnetisch markiert werden. Das magnetische Element zieht diese an, wenn die markierten Mikroorganismen in die Reichweite des magnetischen Feldes des magnetischen Elementes gelangen. Durch das Verfahren können Mikroorganismen schnell und einfach mittels einem Ligand magnetisch markiert oder dekoriert werden und mit dem magnetischen Element des Biopsie-Instrumentes gebunden werden. Hierdurch ist es beispielsweise möglich, mikrobielle Erreger aus dem Blutkreislauf anzureichern und das Resistenzmuster zu bestimmen. Dieses Verfahren wird dadurch ermöglicht, dass bakterielle Krankheitserreger mit magnetischen Nanopartikeln *in vivo* dekoriert werden und sich mit einem magnetischen Element des Biopsie-Instrumentes auch unter nicht-tubulären Strömungsverhältnissen, wie sie im Blutkreislauf vorliegen, anreichern lassen. Vorteilhafterweise ist es möglich, Erreger oder Mikroorganismen in dem Blutkreislauf von Patienten mit Sepsis nachzuweisen und eine schnellere Bestimmung des Resistenzprofiles durchzuführen. Generell sind der Erregemachweis und die Bestimmung seines Resistenzmusters die Voraussetzung, um eine gezielte und ökonomische Antibiotikatherapie zu gewährleisten.

Bevorzugt ist, dass der Ligand Oligoacyllysin und/oder ein Glykopeptid-Antibiotika ist. Das mimetische Lipopeptid Oligoacyllysin (NC(12)-2-beta(12)), das an ein breites Spektrum von Bakterienzellen in verschiedenen Medien schnell und mit hoher Affinität bindet, wird als bevorzugter Ligand genutzt. Im Gegensatz zu natürlichen Peptiden wird das synthetische Oligoacyllysin nicht enzymatisch abgebaut und hat eine Halbwertszeit im Blut von mehreren Stunden. Es muss daher nur in geringen Konzentrationen eingesetzt werden und ist nachweislich nicht toxisch und nicht hämolytisch. Da Oligoacyllysin nicht bakterizid wirkt, können Keime lebend isoliert und im Labor angezüchtet werden. Oligoacyllysin kann vorteilhafterweise chemisch kovalent an magnetische Nanopartikel gekoppelt werden. Ein anderer möglicher Ligand stammt aus der Gruppe der Glykopeptid-Antibiotika, bevorzugt das Glykopeptid Vancomycin. Vancomycin bildet eine nicht-kovalente Bindung mit den beiden endständigen D-Alanyl-D-Alanyl-Resten der Aminosäureseitenketten der Zellwand-Grundbausteine (N-Acety-Glucosamin-N-Acetyl-N-Muraminsäure-Pentapeptid) aus. Es bindet mit hoher Avidität an grampositive Bakterien und mit etwas geringerer Avidität auch an gramnegative Keime. Es ist überraschenderweise möglich, mittels dem Ligand Mikroorganismen zu dekorieren, wobei diese durch den kovalent gebundenen magnetischen Nanopartikel ebenfalls magnetisch werden. Hierdurch ist eine Bindung und Anreicherung derartig dekorierter Mikroorganismen mittels dem magnetischen Element des Biopsie-Instrumentes möglich. Es war völlig überraschend, dass das magnetische Element nicht die Nachteile und Mängel der im Stand der Technik beschriebenen Biopsiegeräte aufweist, die vollständig magnetisch sind. Es hat sich herausgestellt, dass das Biopsie-Instrument kleiner ausgeführt werden kann, wodurch es in Bereiche gelangt, die zuvor für ein Biopsiegerät nicht zugänglich waren. Es ist somit möglich, mit dem bevorzugten Biopsie-Instrument mikrobielle Erreger oder Mikroorganismen, die beispielsweise bei einer Sepsis vorhanden sind, auf zu spüren, anzureichern und zu isolieren.

Für den kundigen Fachmann ist es leicht ersichtlich, dass magnetische Partikel auch mit anderen Liganden ausgestattet werden können, die aus der Gruppe umfassend Antikörper, Antikörperfragmente, synthetische Protein-Scaffolds, Peptide, Nukleinsäuren, Rezeptoren und/oder anorganischen Materialien ausgewählt sein können, um andere Zielobjekte innerhalb des Körpers zu markieren, um danach mit Hilfe des magnetischen Biopsie-Instrumentes im Körper angereichert zu werden und sodann für weitere Zwecke aus dem Körper gewonnen werden können. Beispiele hierfür sind die Markierung von Tumorzellen, Trophoblasten, Biomarkern oder anderen niedrig konzentrierten Zellpopulationen oder Molekülen.

Es ist weiterhin Gegenstand der Erfindung, einen Biopsie-Kit zur Anreicherung von Probenmaterial aus unterschiedlichen Körperregionen und Körperhohlsystemen und dessen Verwendung zur Verfügung zu stellen. Das Biopsie-Kit umfasst mindestens die folgenden Komponenten des Biopsie-Instrumentes:
i. ein Führungselement, umfassend einen feder-elastischen distalen und einen proximalen Bereich,
ii. ein oder mehrere magnetische Elemente, welche zwischen dem distalen und dem proximalen Bereich des Führungselement angebracht sind, wobei auf der Oberfläche von mindestens einem magnetische Element Detektionsmoleküle aufgebracht sind, und
iii. ein mit dem proximalen Bereich des Führungselementes verbundenes Stabilisierungselement,
wobei das Führungselement, das magnetische Element und das Stabilisierungselement sequentiell von distal nach proximal angeordnet sind.

Das Biopsie-Kit umfasst mindestens ein Führungselement, ein magnetisches Element und ein Stabilisierungselement, wobei das Führungselement, das magnetische Element und das Stabilisierungselement sequentiell von distal nach proximal angeordnet sind. Das Führungselement weist einen distalen und einen proximalen Bereich auf und der distale Bereich ist feder-elastisch gestaltet. Es kann ebenfalls vorteilhaft sein, Sonderformen des Biopsie-Instrumentes zu konstruieren, bei denen das magnetische Element zwei oder mehr magnetische Teilelemente mit unterschiedlichen Detektionsmolekülen umfasst und zwischen dem distalem und proximalem Anteil des Führungselementes angeordnet ist. Es kann auch bevorzugt sein, wenigstens ein magnetisches Element ohne Detektionsmoleküle bereitzustellen. Vorteilhafterweise kann die Funktionalisierung des distalen Anteiles des Führungselementes mit Detektionsmolekülen erfolgen und die feder-elastischen Materialeigenschaften dieser Region erhalten bleiben. Ergänzt werden kann das Biopsie-Kit um spezifische Komponenten, die bevorzugt die Anwendung für spezielle Biopsie-Orte erleichtern (beispielsweise im Blutgefäßsystem oder im Urogenitalapparat) oder eine Anwendung unter Nutzung anderer medizintechnischer Hilfsmittel (z.B. von starren oder flexiblen Endoskopen) ermöglichen.

Die Verletzungsgefahr des Patienten bei der Biopsie wird durch das Biospie-Kit, genauer durch die Gestaltung des Biopsie-Kit reduziert. Besonders der feder-elastische distale Bereich des Führungselementes stellt sicher, dass bei Kontakt mit einer Oberfläche, wie z.B. eine Gefäßwand oder ein Organ, keine Beschädigung des biologischen Materials erfolgt. Das feder-elastische Führungselement verformt sich, sobald der Druck auf die Oberfläche zu groß ist, wodurch der Druck auf die Oberfläche reduziert wird.

Zwischen dem distalen und dem proximalen Bereich des Führungselementes ist mindestens ein magnetische Element angebracht, das insbesondere der magnetischen Bindung von magnetischen Nanopartikeln, magnetisierten Mikroorganismen oder magnetisierten Liganden dient. Somit kann bedingt durch die spezielle Ausgestaltung des magnetische Elements eine Anreicherung von speziellen Zellen oder Molekülen, bevorzugt mikrobiellen Erregem im Körper von Mensch und Tier, d.h. *in situ,* erfolgen, was eine Abkehr vom technisch Üblichen darstellt und ein lang bestehendes Problem des Stands der Technik löst. Bedingt durch die hohe Spezifität des magnetische Elements wird eine schnelle Anreicherung von Probenmaterial erreicht, wodurch der Patient geschont und entlastet wird. Außerdem kann die Anreicherung in System erfolgen, die durch eine hohe Strömung gekennzeichnet sind.

Des Weiteren ist ein Stabilisierungselement mit dem proximalen Bereich des Führungselementes verbunden. Das Stabilisierungselement dient der Fixierung des magnetische Elements während der Biopsie und verhindert so eine Dislokation dessen. Somit wird erreicht, dass das Biopsie-Instrument als Ganzes aus dem Patienten entfernt wird, d.h. keine Komponente des Biopsie-Instrumentes interagiert mit Oberflächen derart, dass eine Beschädigung der Oberflächen verursacht wird und Komponenten des Instrumentes in den Körperregionen verbleiben. Vorteile des erfindungsgemäßen Biopsie-Kit sind die Genauigkeit mit der das Instrument in Körperregionen positioniert werden kann und die Spezifität und Sensitivität mit der sich Zellen und Moleküle *in situ* anreichern lassen. Insbesondere können magnetisierte Mikroorganismen angereichert werden, die ggf. mit einer Krankheit assoziiert sind. Das Biopsie-Kit ist leicht von einem Fachmann bedienbar und reduziert das Verletzungsrisiko für den Patienten. Weitere Vorteile sind die geringen Kosten des Biopsie-Kits und eine hohe Flexibilität bei der Ausgestaltung für unterschiedliche Biopsieorte und Anwendungsfälle auch unter Nutzung zusätzlicher Hilfsmittel wie z.B. Endoskopen. Des Weiteren ist eine schnelle und verlässliche Anreicherung von Proben zur Analyse für die Diagnose von Krankheiten ein entscheidendes Kriterium.

Es wird ein Biopsie-Kit bereitgestellt mit dem es gelingt, die für eine Probenanreicherung notwendigen Komponenten zu positionieren, stationär für die zur Anreicherung des spezifischen Probenmaterials erforderlichen Zeitspanne im Körper zu halten und nach der Verweilzeit im Körper mit dem gesammelten Probenmaterial in Gänze zu entfernen. Zur korrekten Positionierung für die Probensammlung und für die erfolgreiche Entnahme Prozedur des Probenmaterials aus dem Körper, dienen visuelle oder andere Führungsmarken an den Komponenten des Biopsie-Kits, die durch Gravur, farbliche oder taktile oder signalgebende Marken realisiert werden können und eine geografische Ortung des Biopsie-Instrumentes während der Positionierung und Probensammlung innerhalb des Körpers und für den Vorgang der Entfernung des Biopsie-Instrument aus dem Körper ermöglichen. Die Positionierung des Biopsie-Instrumentes kann mittels bildgebender Verfahren in situ sichergestellt werden. Hierzu kann der Fachmann bekannte kontrastgebende Mittel konstruktionsseitig verwenden. Auch kann das Biopsie-Kit hinsichtlich der Dimensionierung der Einzelkomponenten für spezielle Biopsie-Orte angepasst werden und um Hilfsmittel ergänzt werden, um für spezielle Anwendungsfälle notwendige oder wünschenswerte Bestandteile während der Biopsie verfügbar zu machen. Des Weiteren kann das Biopsie-Kit als Einmal-Artikel bereitgestellt werden, welches hygienischen Ansprüchen in medizinischen Einrichtungen besser erfüllt.

Das Biopsie-Kit kann vorteilhafterweise zur Entnahme von Proben vorzugsweise aus dem Blutgefäßsystem unter Einbeziehung der Herzkammern, des Lungen- und Körperkreislaufes und des arteriellen und venösen Systems als spezielle Biopsieorte, dem Gastrointestinaltrakt, wobei das Biopsie-Instrument wahlweise von oral oder anal positioniert werden kann, aus den ableitetenden Drüsengängen von Bauchspeicheldrüse, Tränendrüse, Ohrspeicheldrüse, aus den ableitetenden Drüsengängen von mükösen Drüsen, von gemischten Drüsen und von Haut- und Hautanhangsdrüsen einschließlich der Milchdrüse, aus dem Spinalkanal oder Himkammersystem, aus der Gallenblase und ihren ableitenden anatomischen Strukturen, aus den ableitenden Hamwegen oder dem lymphatischen System oder zur Entnahme von Proben aus den Körperhöhlen des Bauches oder des Brustkorbes, der Gebärmutter, des Urogenitalapparates oder eines Gelenkspaltes verwendet werden.

Eine flexible Gestaltung des Biopsie-Kits erlaubt die Anreicherung von Proben in unterschiedlichen Systemen. Ein bevorzugtes Anwendungsgebiet ist das Blutgefäßsystem des Körpers. In diesem Fall wird das Biopsie-Kit über eine Blutgefäß-Verweilkanüle im Gefäßsystem positioniert. Weitere Anwendungsgebiete umfassen den Gastrointestinaltrakt, die ableitenden Gallengänge, den *ductus pancreaticus* oder die ableitenden Harnwege. Während für die Anwendung im Blutgefäßsystem der Außendurchmesser des Biopsie-Kits sich an dem Durchmesser des Blutgefäßes orientiert ist die Länge abhängig von der Punktionsstelle und der Positionierung für die Probensammlung *in situ.* Der Außendurchmesser bei Platzierung im *ductus pancreaticus, ductus choledochus, ductus cysticus* oder den ableitenden Hamwegen oder der Harnblase oder dem Peritoneum oder dem Tracheobronchialbaum oder der Pleurahöhle oder einem Gelenkspalt kann entsprechend angepasst werden. Die vorteilhafte Ausführungsform und Dimensionierung kann somit durch schnelle und einfache Adaptationen von dem Fachmann vorgenommen werden. Es löst demgemäß ein lange im Stand der Technik bestehendes Problem und stellt eine schnelle und spezifische Ausführungsform zur Verfügung, die in unterschiedlichen Körperregionen vorzugsweise in Körperhohlsystemen einsetzbar ist und die Nachteile des Stands der Technik nicht aufweist.

Ausführungsbeschreibungen des Biopsie-Kits erfolgen beispielhaft für Anwendungsfälle unter Nutzung eines Endoskops und für die Probenanreicherung und Entnahme aus dem Blutgefäßsystem als Hauptanwendungsfälle, die Zusammenstellung des Biopsie-Kits ist aber auf diese Einzelfälle nicht beschränkt, sondern kann durch den kundigen Fachmann für andere Anwendungsfälle geändert oder ergänzt werden.

Vorteilhaft ist die Verwendung eines Biopsie-Kits, wenn das Biopsie-Instrument über endoskopische Hilfsmittel, vorzugsweise Gastroduodenoskop, Cystoskop, Urteroskop, Rektoskop, Proktoskop, Coloskop, Arthroskop, Laparoskop, Kolposkop, Hysteroskop, Ophthalmoskop, Laryngoskop und/oder Bronchoskop platziert wird. Ein Endoskop ist im Sinne der Erfindung ein Gerät, mit dem das Innere von lebenden oder toten Organismen, untersucht oder manipuliert werden kann. Für den Gebrauch des Biopsie-Instrumentes über ein Endoskop sind folgende Bestandteile als Komponenten des Biopsie-Kits vorteilhaft:
i. ein Führungselement, umfassend einen feder-elastischen distalen und einen proximalen Bereich,
ii. ein oder mehrere Detektionsmoleküle aufweisende magnetische Elemente, welche zwischen dem distalen und dem proximalen Bereich des Führungselement angebracht ist,
iii. ein mit dem proximalen Bereich des Führungselementes verbundenes Stabilisierungselement,
iv. ein Adapter, der das Einbringen des Biopsie-Instrumentes über den Arbeitskanal des Endokops ermöglicht und/oder
v. eine transparente und longitudinal verschiebbare Hülle, die auf den Adapter aufsteckbar ist, das Biopsie-Instrument außerhalb des Körpers umgibt und eine hygienische Handhabung während seiner Verweilzeit im Körper gewährleistet.

Die Länge des gesamten Biopsie-Kits, umfassend das Führungselement, das magnetische Element und das Stabilisierungselement, ist so zu wählen, dass nach endoskopischer Platzierung, in der Regel über den Instrumentenkanal des Endoskops, das Endoskop aus dem Körper entfernt werden kann, ohne die Lage des Biopsie-Kits zu verändern. Dieses lässt sich dadurch bewerkstelligen, dass die Gesamtlänge des Biopsie-Kits an die Gesamtlänge des Endoskopes angepasst wird. Einem kundiger Fachmann ist es leicht möglich Sonderformen des Biopsie-Instrumentes zu konstruieren, bei denen das magnetische Element zwei oder mehr magnetische Teilelemente mit unterschiedlichen Detektionsmolekülen umfasst und zwischen distalem und proximalem Anteil des Führungselementes angeordnet ist oder die Funktionalisierung des distalen Anteiles des Führungselementes mit Detektionsmolekülen vorzunehmen unter Erhalt der federelastische Materialeigenschaften dieser Region.

Vorteilhaft ist die Verwendung eines Biopsie-Kits, wenn das Biopsie-Instrument über einen Gefäßzugang in ein Gefäßsystem eingebracht wird. Hierbei können die auf dem magnetischen Element angebrachten Detektionsmoleküle verschiedene Moleküle und/oder Zellen im Gefäßsystem detektieren. Für den Gebrauch des Biopsie-Kits über einen Gefäßzugang sind folgende Bestandteile als weitere Komponenten des Biopsie-Kits sinnvoll:
i. eine Blutgefäß-Verweilkanüle mit Punktionsnadel;
ii. ein Adapter, der sich an die Blutgefäß-Verweilkanüle anbringen und über den sich das Biopsie-Instrument intravasal positionieren lässt;
iii. einen Port, der vorzugsweise am Adapter ausgeführt ist und über den während der Verweilzeit des Biopsie-Instrumentes im Körper eine Infusionslösung appliziert werden kann und/oder
iv. eine transparente und verschiebbare Hülle, die sich auf den Adapter aufschiebbar ist, das Biopsie-Instrument außerhalb des Körpers umgibt und eine hygienische Handhabung während seiner Verweilzeit im Körper gewährleistet.

Durch die vorteilhafte Ausführungsform kann schnell und effizient eine Probenanreicherung mit einer hohen Ausbeute erreicht werden, wenn das Biopsie-Kit in ein Gefäßsystem über einen Gefäßzugang eingeführt wird. Dies gilt insbesondere dann, wenn der Kit alle genannten Bestandteile umfasst. Die gesammelte Probe kann nach Ausfuhr aus dem Gefäßsystem direkt analysiert werden oder an entsprechende Bearbeitungsstellen weitergeleitet werden. Die Ausführungsform stellt somit eine erhebliche Arbeitserleichterung für Krankenhauspersonal dar.

In sehr ähnlicher Weise kann das Biopsie-Kit zur Anreicherung und Sammlung von Untersuchungsmaterial aus dem Spinalkanal und dem Periduralraum genutzt werden, wenn anstelle der Blutgefäß-Verweilkanüle eine Spinalkanal-Punktionskanüle bzw. eine Kanüle zur Punktion des Periduralraums genutzt wird, wie sie beispielsweise bei der Periduralanästhesie zum Einsatz kommt.

Neben der Platzierung über eine Blutgefäß-Verweilkanüle und via Endoskop ist auch eine Platzierung im Rahmen eines operativen Eingriffes oder im Rahmen einer minimal-invasiven chirurgischen Maßnahme vorteilhaft, was vorteilhafterweise an die Anforderungen bezüglich der Dimensionierung des Biopsie-Instrumentes keine zusätzlichen Anforderungen stellt als die bereits genannten. Die vorteilhafte Ausführungsform führt zu einer Verbesserung der Qualität und zu der Erhöhung der Zuverlässigkeit der Probenanreicherung.

Es ist bevorzugt, wenn das Biospie-Instrument und/oder das Kit zur Anreicherung von Proben verwendet wird und die Proben bevorzugt zur Diagnose insbesondere pränatalen Diagnose, Krebsdiagnose, der Therapie-Verlaufskontrolle (point of care Diagnostik) und/oder zur Diagnose der Homöostase von Patienten verwendet werden. Ferner lässt sich das Biopsie-Instrument vorteilhafterweise zur Diagnose einer Erkrankung ausgewählt aus der Gruppe umfassend Erberkrankungen, proliferativen Erkrankungen, inflammatorischen Erkrankungen, Autoimmunerkrankungen, Infektionserkrankungen, hormonellen Erkrankungen, Erkrankungen des Blutes und der blutbildenden Organe, Erkrankungen des Verdauungstraktes, der Leber, der Galle, der Bauchspeicheldrüse, Erkrankungen des Urogenitaltraktes und der Niere, Erkrankungen des Herzens, krankhafte Veränderungen des Blutgefäßsystems und des Lymphsystems, Erkrankungen der Lunge, Erkrankungen des zentralen oder peripheren Nervensystems sowie der elektrischen Reizweiterleitung und neurodegenerative Erkrankungen benutzen. Das Biopsie-Instrument sammelt vorteilhafterweise Proben, die zur Diagnose der genannten Krankheiten genutzt werden können, das heißt das Biopsie-Instrument und das mit diesem realisierte Verfahren dient der Gewinnung eines Zwischenschrittes auf dem Weg zur Diagnosestellung. Das Biopsie-Instrument kann somit zur Diagnose von unterschiedlichen Erkrankungen herangezogen werden. Überraschenderweise kann das Biopsie-Instrument zur Diagnose oder zur Erzielung eines Zwischenschrittes auf dem Weg zur Diagnosestellung und/oder zur Kontrolle des Therapieverlaufs folgender Krankheiten eingesetzt werden:
- Erbkrankheiten bevorzugt umfassend autosomal rezessive, autosomal dominante, gonosomale und mitochondriale beziehungsweise extrachromosomale Erberkrankung und/oder eine Erkrankung, die auf eine genetische Disposition zurück geführt werden kann. Proliferative Krankheiten umfassen bevorzugt Tumore, Präcancerose, Dysplasie, neuroendokrine Tumore, Endometriose und/oder Metaplasie.
- Autoimmunerkrankungen bevorzugt umfassend rheumatoide Arthritis, inflammatorische Darmerkrankung, Osteoarthritis, neuropathische Schmerzen, Alopecia areata, Psoriasis, psoriathrische Arthritis, akute Pankreatitis, Allograftabstoßung, Allergien, allergische Entzündungen der Lunge, Multiple Sclerose, Alzheimer, Morbus Crohn und/oder systemischer Lupus erythematous.
- Infektionskrankheiten bevorzugt umfassend durch parasitäre Erkrankung, bakterielle Erkrankung und/oder viralen Erkrankungen ausgelöste Infekte. Es war völlig überraschend, dass mittels des Biopsie-Instrumentes Mikroorganismen schnell und sicher gesammelt und identifiziert werden können. Hierbei werden die Mikroorganismen bevorzugt durch Liganden, die mit magnetischen Nanopartikeln gekoppelt sind, erkannt, wobei die Liganden an die Mikroorganismen binden. Die Mikroorganismen werden so magnetisch dekoriert und können mittels dem magnetischen Element des Biopsie-Instrumentes gebunden werden.
- Hormonelle Erkrankungen bevorzugt umfassend Erkrankung des Zuckerstoffwechsels, des Fettstoffwechsels, des Proteinstoffwechsels, der sexuellen Entwicklung und Fortpflanzung, des Wasser-Salz-Haushaltes, des Wachstums und/oder der Zellbildung.

Die Verweildauer des Biopsie-Instrumentes in den Körperregionen kann bevorzugt variiert werden und ist von den vorliegenden Konzentrationen des anzureichemden Materials abhängig. Das Biopsie-Instrument kann so in den Körperregionen positioniert werden, dass eine spezifische Anreicherung möglich ist. Mittels des magnetischen Elementes können bestimmte Mikroorganismen, die vorteilhafterweise mit magnetischen Nanopartikeln oder sonstigen magnetischen Elementen markiert sind, einfach und schnell detektiert und/oder isoliert werden. Dies stellt einen Fortschritt dar, da so eine schnelle und verlässliche Diagnose und Therapie-Verlaufskontrolle möglich ist. Nach Anreicherung der Probe kann das magnetische Element bevorzugt mittels einer Abdeckvorrichtung bedeckt, wodurch ein Verlust der Probe während der Entnahme aus dem Körper und eine Kontamination außerhalb des Körpers verhindert wird. Durch die Verwendung des Biopsie-Instrumentes werden Fehler bei der Probenentnahme verhindert und eine hohe Spezifität sichergestellt. Des Weiteren kann es für die Anreicherung von Proben verwendet werden, die in geringen Konzentrationen in schwer zugänglichen Körperregionen vorhanden sind.

Durch den einfachen und schnellen Austausch des magnetischen Elements oder der Detektionsmoleküle des magnetischen Elements, kann das Biopsie-Instrument in verschiedenen medizinischen Bereichen zur Diagnostik eingesetzt werden. Gerade bei der pränatalen und Tumor-Diagnostik ist eine schnelle und verlässliche Diagnostik entscheidend. Frühzeitig erkannt, können Krankheiten wie Herzfehler, Spina bifida und Lippen-Kiefer-Gaumenspalten therapiert werden. Somit bietet die Verwendung des Biopsie-Instrumentes mit den entsprechenden Detektionsmolekülen eine Möglichkeit, Proben anzureichern und diese auf Krankheiten zu analysieren und den Effekt einer eingeschlagenen Therapie zu überwachen, zu prüfen und gegebenenfalls zu ändern. Auch in dem Bereich der Krebsdiagnostik ist eine schnelle und zuverlässige Detektion unabdingbar, um frühzeitig therapeutische Behandlungen zu beginnen, zu überwachen und gegebenenfalls anzupassen. Hierfür muss jedoch der Tumor identifiziert werden. Die Verwendung des Biospie-Instrumentes offenbart die Möglichkeit, Krebsmarker, die charakteristisch für bestimmte Tumorarten sind, in Körperregionen, z.B. dem Gefäßsystem oder ableitenden Drüsengängen anzureichern und so eine schnelle Diagnose und Therapie-Verlaufskontrolle zu ermöglichen. Vorteilhafterweise kann das Biopsie-Instrument zur Therapie-Verlaufskontrolle benutzt werden, das heißt es werden Proben mit dem Biopsie-Instrument gesammelt, die analysiert werden und der Kontrolle des Therapie-Verlaufs dienen. Diese umfasst z. B. die Überwachung einer Tumorbehandlung, die Überwachung einer autologen, syngenen, allogenen, xenogenen oder alloplastischen Transplantation, der Überwachung einer entzündlichen Erkrankung, der Überwachung einer Infektionserkrankung, der Überwachung einer hormonellen Erkrankung, der Überwachung einer psychiatrischen Erkrankung und/oder der Überwachung einer neuro-degenerativen Erkrankung. Überraschenderweise kann das Biopsie-Instrument einfach und zuverlässig an die Diagnose von unterschiedlichen Krankheiten angepasst werden. Beispielsweise kann das magnetische Element mit Fängerstrukturen versehen werden. Die Fängerstrukturen können im Sinne der Erfindung auch als Detektionsmoleküle bezeichnet werden. Dem Fachmann sind diese Fängerstrukturen bekannt wodurch ebenfalls die Auswertung der gesammelten Proben bevorzugt automatisiert erfolgen kann. Die Proben sind demgemäß innerhalb kurzer Zeit ausgewertet und die gesammelten Daten können vorteilhafterweise zur Entscheidung über die Weiterbehandlung herangezogen werden.

Besonders vorteilhaft kann das Instrument zur Primärdiagnostik, zur Diagnostik der Tumorausbreitung oder zum Tumor Grading eingesetzt werden. Es können vorteilhafterweise Risikopatienten über einen längeren Zeitraum und in regelmäßigen Zeitintervallen Proben entnommen werden, um so ggf. eine Entstehung eines Tumors frühzeitig zu detektieren. Beispielsweise können die gesammelten Biopsie-Proben von einem Labor analysiert und ausgewertet werden. Jedoch kann das Instrument nicht nur zur Primärdiagnostik verwendet werden, sondern auch zur Langzeitüberwachung der Tumorausbreitung. Es kann beispielsweise das Tumorwachstum oder die Metastasenbildung eines Tumors überwacht werden. Diese Daten in Zusammenhang mit beispielsweise dem Differenzierungsgrad (Tumor Grading) des Tumors geben vorteilhafterweise dem behandelten Arzt wichtige Informationen, die für den weiteren Behandlungsverlauf entscheidend sein können. Vorteilhafterweise können die gesammelten Proben schnell und zuverlässig analysiert werden, was für eine erfolgreiche Behandlung ausschlaggebend ist.

Vorteilhafterweise kann das Biopsie-Instrument zur pränatalen Diagnose genutzt werden. Hierbei werden Proben gesammelt und bevorzugt auf Genmutationen, Chromosomenmutationen und Chromosomenaberrationen aus der Gruppe umfassend Deletion, Inversion, Duplikation, Translokation, Ringchromosomen, und/oder eine Störung der Gen-Transkription, der Gen-Translation, der mRNA-Stabilität, einer Splice-Varianten, einer Störung des mRNA Transportes ins Cytoplasma, der Protein-Biosynthese und/oder eines epigenetischen Faktors. Es können vorteilhafterweise Detektionsmoleküle, sogenannte Fängermoleküle, auf der Oberfläche eines oder mehrerer magnetischer Elemente angebracht werden. Die Moleküle sind bevorzugt spezifisch für die zu detektierenden Proben, die ebenfalls mit magnetischen Nanopartikeln markiert werden können. Die gesammelten Proben können beispielsweise in einem Labor weitergehend analysiert werden und einem behandelnden Arzt zugehen. Vorteilhafterweise ist durch die Anreicherung von bevorzugt ausschließlich spezifischen Proben die Analyse wesentlich vereinfacht und in kürzerer Zeit durchführbar. Das Biopsie-Instrument ermöglicht nicht nur eine schnellere Analyse, sondern verbessert ebenfalls die Qualität der Proben. Es werden bevorzugt spezifische Proben gesammelt, die vorteilhafterweise bei dem Ausführen des Instrumentes aus dem Körper vor einer Dislokation geschützt sind.

Des Weiteren kann das Biopsie-Instrument dazu verwendet werden, die Homöostase von Patienten zu verfolgen. Gerade bei schwerwiegenden Krankheiten oder nach instrumentellen, chirurgischen Eingriffen ist eine kontinuierliche Überwachung des Patientenzustandes erforderlich. Das Biopsie-Instrument kann hierfür universell eingesetzt werden und vorteilhafterweise an die unterschiedlichen Anforderungen einfach, schnell und kostengünstig angepasst werden.

Weitere Vorteile des erfindungsgemäßen Biopsie-Instrumentes sind: Verbilligung, Vereinfachung, Ersparnis an Zeit, Material, Arbeitsstufen, Kosten oder schwer beschaffbaren Rohstoffen, erhöhte Zuverlässigkeit, Beseitigung von Fehlern, Qualitätshebung, Wartungsfreiheit, größere Effektivität, höhere Ausbeute, Vermehrung technischer Möglichkeiten, Eröffnung eines neuen Gebiets, erstmalige Lösung einer Aufgabe, Möglichkeit der Rationalisierung und/oder Miniaturisierung.

Die Erfindung soll im Folgenden anhand eines Beispiels näher erläutert werden, ohne jedoch auf das Beispiel begrenzt zu sein.

### Festlegung der Dimensionierung und Auswahl der Magnetwerkstoffe für die Herstellung von Eichmagneten und Charakterisierung ihrer magnetischen Eigenschaften

Magnete können beliebig dimensioniert oder konstruiert werden. Allerdings sind die anatomischen Verhältnisse innerhalb des Körpers limitierend. Da in einer bevorzugten Ausführungsform miniaturisierte Festmagnete genutzt werden, die über eine ausreichend starke Anziehungskraft verfügen, werden Eichmagnete aus verschiedenen Magnetwerkstoffen (z.B. Neodym, Samarium-Kobalt) gefertigt. Desweiteren können aber auch Elektromagnete verwendet werden, die sich durch ihre Dimensionierung an die anatomischen Verhältnisse innerhalb des Körpers anpassbar sind und den Vorteil besitzen, dass ihr Magnetfeld an- und abgeschaltet werden kann.

### Identifizierung von Liganden und Koppelung dieser an magnetische Nanopartikel

Das mimetische Lipopeptid OligoAcylLysin (NC(12)-2-beta(12)), das an ein breites Spektrum von Bakterienzellen in verschiedenen Medien schnell und mit hoher Affinität bindet, kann als ein bevorzugter Ligand genutzt werden. Im Gegensatz zu natürlichen Peptiden wird das synthetische OligoAcylLysin nicht enzymatisch abgebaut und hat eine Halbwertszeit im Blut von mehreren Stunden. Es muss daher nur in geringen Konzentrationen eingesetzt werden und ist nachweislich nicht toxisch und nicht hämolytisch. Da OligoAcylLysin nicht bakterizid wirkt, können Keime lebend isoliert und im Labor angezüchtet werden. OligoAcylLysin kann chemisch kovalent an magnetische Nanopartikel gekoppelt werden. Ein anderer bevorzugter Ligand für verschiedene Mikroorganismen stellt das Glykopeptid Vancomycin dar. Vancomycin bildet eine nicht-kovalente Bindung mit den beiden endständigen D-Alanyl-D-Alanyl-Resten der Aminosäureseitenketten der Zellwand-Grundbausteine (N-Acety-Glucosamin-N-Acetyl-N-Muraminsäure-Pentapeptid) aus. Es bindet insbesondere mit hoher Avidität an grampositive Bakterien und mit etwas geringerer Avidität auch an gramnegative Keime.

Als Negativkontrolle für die beiden Liganden-gekoppelten und somit funktionalisierten Nanopartikel können bevorzugt dextranumhüllte Nanopartikel eingesetzt werden, die keine Funktionalisierung aufweisen.

### Magnetische Nanopartike

Unterschiedlich große Eisenoxidpartikel mit unterschiedlicher Hülle können zum Einsatz kommen und mit unterschiedlichen Liganden ausgestattet werden. Ein Partikeltyp eignet sich in besonderer Weise auch für das magnetic particle imaging. Dieser Partikeltyp wird bevorzugt für die Ligandenkopplung eingesetzt. Die Partikel können hinsichtlich Größe und Ladung charakterisiert werden, wobei das magnetische Relaxationsverhalten und die magnetische Suszeptibilität bestimmt und die kolloidale Stabilität geprüft werden kann.

### Nanopartikelherstellung und -funktionalisierung

Die Syntheseprotokolle für die Herstellung magnetischer Nanopartikel können Präzipitationsverfahren in wässriger Lösung oder auch Assoziationsreaktionen in organischen Lösungsmitteln umfassen. Das Hüllmaterial kann simultan oder sequentiell aufgebracht werden. Die Ligandenkopplung an funktionelle Gruppen der Partikelhülle kann über Carboxyl-, Amino, Thiol- oder andere in der Biochemie gängigen Bindungsstellen erfolgen aber auch über dick Reaktionen, die nicht natürliche Aminosäuren nutzen durchgeführt werden, um ausgewählte Liganden für die Dekoration von Modellbakterien zu optimieren.

### Identifizierung, Züchtung und Charakterisierung von klinisch relevanten Modellbakterien

Um die Wirkungsweise des Biopsie-Instrumentes zu testen, können beispielsweise folgende Bakterien eingesetzt werden: *E. coli* (DSMZ-Nr. 1103, ATCC-Nr. 25922); *Staphylococcus aureus* (DSMZ-Nr. 20231, ATCC-Nr. 12600). Diese Stämme werden mit den üblichen mikrobiologischen Verfahren gezüchtet und charakterisiert.

### Magnetotaktische Bakterien

Magnetotaktische Bakterien können ebenfalls als natürlich vorkommende magnetische "Eichbakterien" in einem artifiziellen Kreislaufsystem eingesetzt werden, um die Funktion des Biopsie-Instrumentes zu testen. Diese Bakterien synthetisieren intrazelluläre magnetische Nanopartikel, sogenannte Magnetosomen, die starke magnetische Dipole erzeugen, welche als biologische Kompassnadeln fungieren.

Magnetotaktische Bakterien können beispielsweise von DSMZ bezogen werden (z. B. *Magnetospirillum magnetotacticum*, DSMZ-Nr. 3856, MS-1, ATCC-Nr. 31632 und in Magnetospirillium Flüssigmedium 380 kultiviert. Zuerst können diese Bakterien im Hinblick auf ihre magnetischen Eigenschaften charakterisiert werden, um Anhaltswerte über die Bindungskapazität der Bakterien am Magneten zu erhalten. Mit dem Ferrozin-Test kann beispielsweise der Eisengehalt pro Bakterienzelle quantitativ bestimmt werden. Mit Hilfe der Magnetrelaxometrie kann das magnetische Verhalten der Bakterien gemessen werden.

### Magnetische Separation

Die Bindungseffizienz der mit einem Liganden funktionalisierten Nanopartikel an Modellbakterien kann mit Hilfe der Magnetseparation untersucht werden. Hierzu können Modellbakterien mit den funktionalisierten Nanopartikeln inkubiert und sodann über eine Magnetsäule gereinigt werden. Bei diesem Verfahren lassen sich magnetisch dekorierte Bakterien von nicht dekorierten trennen.

### Kreislaufsystem

Um das Biopsie-Instrument zu testen, steht ein physiologisches Kreislaufsystem zur Verfügung, in dem ein pulsatiles Strömungsprofil in unterschiedlich kalibrierten Röhren mit unterschiedlich elastischem Verhalten erzielt werden kann. Damit können Strömungsverhältnisse simuliert werden, wie sie an unterschiedlichen anatomischen Orten des Blutkreislaufsystems vorliegen.

### Untersuchungen in einem Kreislaufsystem

Hierzu werden in einem physiologischen *in-vitro* Modell des Blutkreislaufsystems magnetotaktische Bakterien appliziert, die vorher charakterisiert wurden. Mit Hilfe von unterschiedlich dimensionierten, miniaturisierten Eichmagneten mit bekannten magnetischen Eigenschaften (Remanenz und Koerzitivstärke) können diese unter physiologischen Strömungsverhältnissen angereichert und isoliert werden. Dabei werden sowohl die Bakterienkonzentration im Medium zu variieren sein als auch die Verweilzeiten des Eichmagneten. In einem ersten Versuchsaufbau wird eine physiologische Pufferlösung im Kreislaufsystem eingesetzt. In einem zweiten Versuchsaufbau werden die magnetischen "Eichbakterien" in gerinnungsgehemmtes Vollblut überimpft und ebenfalls mit miniaturisierten Eichmagneten aus dem artifiziellen Kreislaufsystem isoliert und quantifiziert.

Die Darstellung, dass sich Bakterien aus dem Blutkreislauf mit dem bevorzugten Biopsieverfahren, welches magnetische Elemente nutzt, isolieren lassen, kann in folgendem Experiment in zwei Stufen gezeigt werden:
1. Stufe: Funktionalisierte magnetische Nanopartikel können mit Bakterien im Reagenzglas inkubiert, in ein Kreislaufsystem appliziert und unter Nutzung eines Eichmagneten isoliert werden.
2. Stufe: Zunächst werden Bakterien, dann funktionalisierte magnetische Nanopartikel in ein Kreislaufsystem appliziert. Anschließend wird ein Eichmagnet in das Kreislaufsystem eingeführt und nach definierten Zeitabständen auf Isolation der Modellbakterien überprüft.

Dieses Experiment kann in physiologischer Pufferlösung und in gerinnungsgehemmtem Vollblut als Flussmittel für das Kreislaufsystem durchgeführt werden. Die Konzentration magnetisch dekorierter Modellbakterien in einem physiologischen Kreislaufmodell ist bekannt, um mit einem miniaturisierten Eichmagneten isoliert zu werden. Auch der Zeitraum, wie lange der Eichmagnet im Kreislaufsystem deponiert sein muss, um eine effektive Bakterienisolation zu ermöglichen, ist bekannt.

### Experiment in vivo

Die als klinisch relevant eingestuften Modellbakterien wurden in definierter Zahl (106 bis 108 KbE) intravenös Ratten appliziert. Sodann wurden funktionalisierte magnetische Nanopartikel in den Kreislauf injiziert. Anschließend kann ein Biopsie-Instrument mit einem magnetischen Element im Blutkreislauf deponiert und nach definierten Zeitabständen die Isolation von Modellbakterien überprüfen.

Die Erfindung wird nunmehr anhand von Figuren beispielhaft beschrieben, ohne auf diese beschränkt zu sein. Es zeigen:
- Fig. 1: eine Seitenansicht eines Führungselementes mit einem distalen und proximalen Bereich,
- Fig. 2: eine Seitenansicht eines Führungselementes mit einem zylindrischen magnetischen Element und einem zylindrischen Stabilisierungselement,
- Fig. 3: eine Seitenansicht eines Biopsie-Instrumentes, mit einer Aufnahmevorrichtung für ein halbzylindrisch-geformtes magnetisches Element,
- Fig. 4: eine Seitenansicht eines Biopsie-Instrumentes, mit einer Aufnahmevorrichtung für zwei halbzylindrisch-geformte magnetische Elemente,
- Fig. 5: eine Seitenansicht eines Biopsie-Instrumentes, mit einer stegförmigen Aufnahmevorrichtung für ein spangenförmig-geformtes magnetisches Element,
- Fig. 6: eine Seitenansicht eines Biopsie-Instrumentes, mit einer Aufnahmeeinrichtung für ein folienförmiges magnetisches Element,
- Fig. 7: eine Seitenansicht eines Biopsie-Instrumentes mit einer Abdeckvorrichtung und
- Fig. 8: eine Seitenansicht eines Biopsie-Instrumentes mit einer Abdeckvorrichtung über einem magnetischen Element.

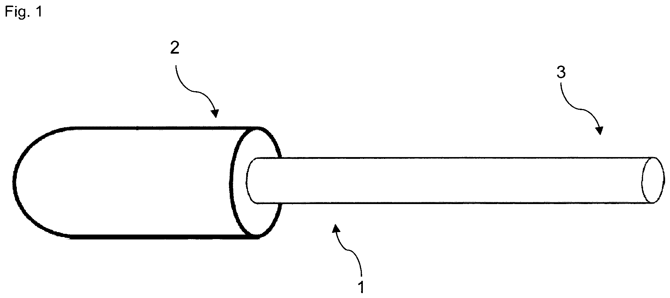

Fig. 1 zeigt eine schematische Seitenansicht eines Führungselementes. Das Führungselement 1 besteht aus einem distalen 2 und proximalen 3 Bereich. Der distale Bereich 2 des Führungselementes 1 ist federelastisch gestaltet. Er kann aus Metallen, vorzugsweise Stahl gefertigt sein. Alternativ können für diese Komponente aber auch nichtmetallische Materialien, aus denen sich dünne zylindrische Strukturen formen lassen, verwendet werden. Polymermaterialien wie z.B. Polytetrafluorethylen kommen hierfür in Frage. Der distale Bereich 2 des Führungselementes 1 ist fest mit dem proximalen Bereich 3 verbunden. Distaler 2 und proximaler 3 Bereich des Führungselementes 1 können aber auch in einem Herstellungsverfahren hergestellt werden, wenn das Material dieses erlaubt was z.B. durch den Einsatz geeigneter Kunststoffe bei der Herstellung möglich ist.

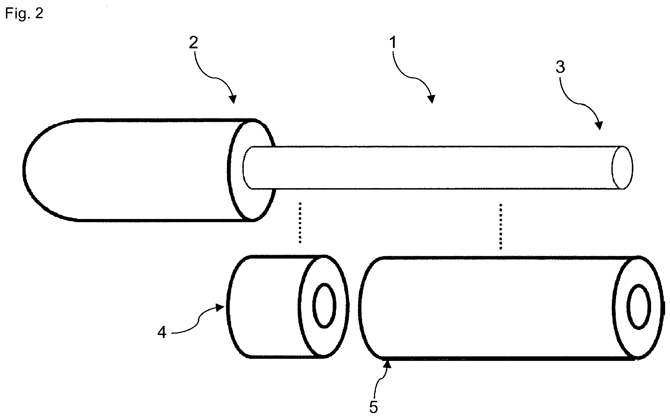

In Fig. 2 ist eine schematische Seitenansicht eines Führungselementes mit einem magnetischen Element und einem Stabilisierungselement gezeigt. Das magnetische Element 4 in Fig. 2 kann zusätzlich Detektionsmoleküle auf seiner Oberfläche tragen, die mit spezifischen Zellen und/oder Molekülen im Körper von Mensch und Tier reagieren und dadurch eine verbesserte Anreicherung von Probenmaterial in situ vor der Entnahme aus dem Körper gestatten. Die Ausgestaltung dieses Elementes ist im Wesentlichen als zylindrischer Hohlkörper auszuführen, der hinsichtlich seiner Abmessung so dimensioniert ist, dass er sich von dem proximalen Bereich 3 auf das Führungselement 1 aufbringen lässt. Hierzu ist der Innendurchmesser dieser Komponente größer als der Außendurchmesser des proximalen Bereiches 3 des Führungselementes 1. Der Außendurchmesser des magnetischen Elements 4 in Relation zum distalen Bereich 2 des Führungselementes 1 kann größer, gleich oder kleiner sein. Vorzugsweise ist der Außendurchmesser des magnetischen Element 4 allerdings 0,01 bis 0,1 mm kleiner als der Außendurchmesser des distalen Bereichs 2 des Führungselementes 1. Materialien zur Herstellung dieser Komponente umfassen Metalle, Kunststoffe oder keramische Werkstoffe, die als Substrat der Verankerung von Detektionsmolekülen dienen können. Auch eine Ausgestaltung in Form von winzigen Hohlfasern oder Schwämmen ist möglich.

Des Weiteren ist in Fig. 2 ein Stabilisierungselement 5 dargestellt. Diese Komponente ist dadurch gekennzeichnet, dass sie rohrförmig ist und sich passgenau auf den proximalen Bereich 3 des Führungselementes 1 aufbringen lässt. Der Außendurchmesser des Stabilisierungselementes 5 entspricht dem Außendurchmesser des distalen Anteils 2 des Führungselementes 1. Die Wandstärke dieses rohrförmigen Elementes ist so dimensioniert, dass das Magnetischen Element 4 rutschfrei durch das Stabilisierungselement 5 auf dem Führungselement 1 fixiert wird. Das Stabilisierungselement 5 und der proximale Bereich 3 des Führungselementes 1 können dazu eine stabile Verbindung eingehen, was z.B. mechanisch, durch Verkleben oder durch Verschweißen dieser Elemente am proximalen Ende des Biopsie-Instrumentes ohne Funktionsverlust realisiert werden kann. Weiterhin ist vorteilhaft, wenn beiden Komponenten reversibel miteinander verbunden werden. Es kann so sichergestellt werden, dass nach erfolgreicher Probenanreicherung das magnetische Element von dem Biopsie-Instrument entfernt werden kann und zur Durchführung von nachgeschalteten Diagnose-Verfahren in die entsprechenden Labore versendet wird. Durch die Verwendung von Materialien für das Stabilisierungselement, die diesem gummielastischen Eigenschaften verleihen, können Biopsie-Instrumente hergestellt werden, die sich optimal an unterschiedliche Biopsie-Orte im Körper platzieren lassen.

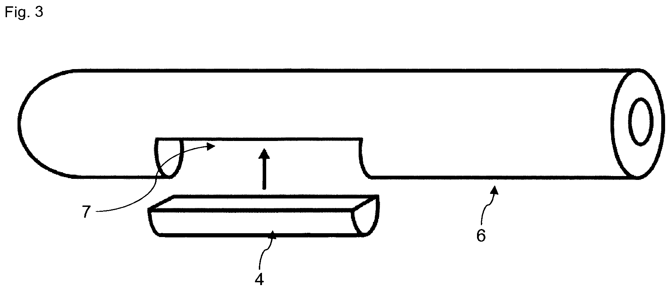

Fig.3 zeigt eine Seitenansicht eines Biopsie-Instrumentes, mit einer Aufnahmevorrichtung für ein halbzylindrisch-geformtes magnetisches Element. Das Führungselement 1 und das Stabilisierungselement 5 können in einem Herstellungsverfahren gefertigt sein und dementsprechend permanent verbunden sein. Das so hergestellte Führungselement/Stabilisierungselement 6 weist eine Aufnahmevorrichtung 7 auf, die der Aufnahme eines magnetischen Elements 4 dient. Die Form der Aufnahmevorrichtung 7 kann unterschiedliche Formen aufweisen, wie z.B. die Form eines Halbzylinders. Das magnetische Element 4 ist entsprechend der Aufnahmevorrichtung 7 geformt. Dies ermöglicht die Variation der zu sammelnden Proben, oder ein schneller und einfacher Austausch des Magnetischen Element es 4, um dieses neuen Gegebenheiten anzupassen.

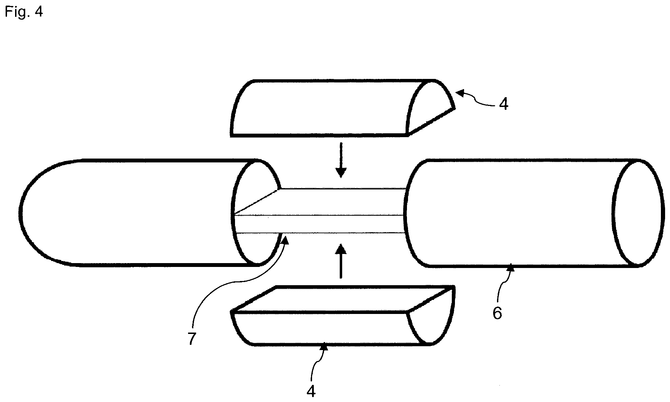

Fig. 4 stellt eine Seitenansicht eines Biopsie-Instrumentes, mit einer Aufnahmevorrichtung für zwei halbzylindrisch-geformte magnetische Elemente dar. Das Biopsie-Instrument kann so gestaltet sein, dass das Führungselement 1 und das Stabilisierungselement 5 aus Fig. 2 in einem Herstellungsverfahren gefertigt sind, ein Führungselement/Stabilisierungselement 6 ergebend und eine Aufnahmevorrichtung 7 aufweisend in die zwei magnetische Elemente 4 eingeführt werden können. Hierbei kann die Aufnahmevorrichtung 7 derart gestaltet sein, dass zweie halbzylindrisch-geformte magnetischen Elemente 4 in diese eingeführt werden können. Auf die beiden magnetischen Elemente 4 können unterschiedliche Detektionsmolekülen aufgebracht sein, die folglich die Anreicherung von unterschiedlichen Zellen und/oder Molekülen erlauben bzw. verbessern. Des Weiteren kann durch eine solche Formgestaltung die Ausbeute der Probe erhöht werden.

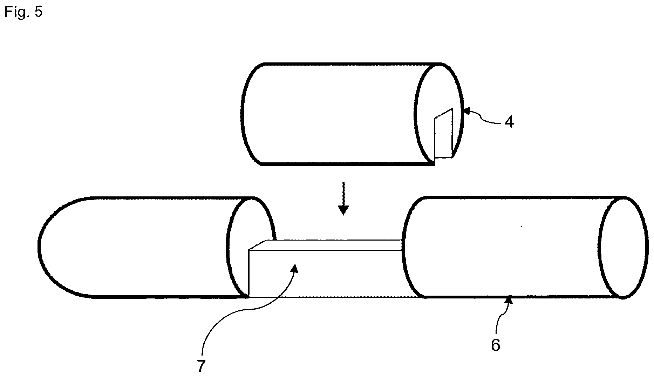

Fig. 5 zeigt eine Seitenansicht eines Biopsie-Instrumentes, mit einer stegförmigen Aufnahmevorrichtung für ein spangenförmig-geformtes magnetisches Element. Die Aufnahmevorrichtung 7 kann so gestaltet werden, dass sie stegförmig geformt ist und dementsprechend ein spangenförmiges magnetisches Element 4 aufnehmen kann. Das so gestaltete magnetische Element 4 kann schnell ausgetauscht werden. Auch bei dieser Ausführungsalternative ist ein magnetisches Element 4 zwischen einem distalen Bereich 2 und einem proximalen Stabilisierungselement 5 angeordnet. Es wird erreicht, dass bei minimaler Verletzungsgefahr für den Patienten die Biofunktion des Instrumentes gewährleistet bleibt und sich das Biopsie-Instrument nach Sammlung von Probenmaterial in situ in Gänze von außen aus dem Körper wieder entfernen lässt.

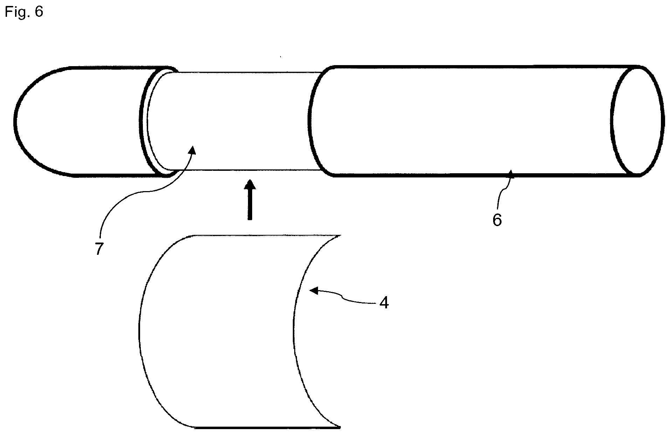

In Fig. 6 ist eine Seitenansicht eines Biopsie-Instrumentes, mit einer Aufnahmeeinrichtung für ein folienförmiges magnetisches Element. Bei Anwendungen, die die Verminderung des Außendurchmessers des Biopsie-Instrumentes verlangen, kann eine folienförmige Aufnahmevorrichtung 7 zur Aufnahme eines folienförmigen magnetischen Elements 4 dienen. Ein folienförmiges magnetisches Element bezeichnet im Sinne der Erfindung eine Form mit einer sehr geringen Dicke und großer Fläche. Besonders in kleinen Gefäßen, in denen Proben angereichert werden müssen, kann diese Ausführungsform genutzt werden. Auch spezielle Detektionsmoleküle, in Form eines anorganischen Materials, mit denen die Anreicherung bestimmter organischer und/oder anorganischen Molekülen erreicht werden kann, können so in die Aufnahmevorrichtung 7 eingebracht werden. So kann das Biopsie-Instrument zur Anreicherung von Zellen und/oder Molekülen dienen, die in geringen Konzentrationen in Körperregionen vorliegen.

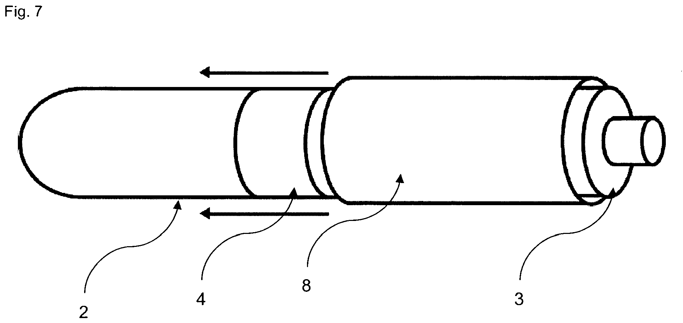

Fig. 7 zeigt eine Seitenansicht eines Biopsie-Instrumentes mit einer Abdeckvorrichtung. Hierbei ist eine Abdeckvorrichtung 8, die eine hülsenförmige Struktur umfassen kann und auf dem Biopsie-Instrument gleitet, dargestellt. Diese Abdeckvorrichtung 8 wird von dem proximalen Bereich des Führungselementes auf das Biopsie-Instrument aufgebracht. Als Materialien für die Abdeckvorrichtung 8 eignen sich Polymere, vorzugsweise Polytetrafluorethylen. Insbesondere wenn die angrenzenden Komponenten aus dem gleichen Material gefertigt sind, wird dadurch eine gute Verschiebbarkeit der Einzelkomponenten erreicht.

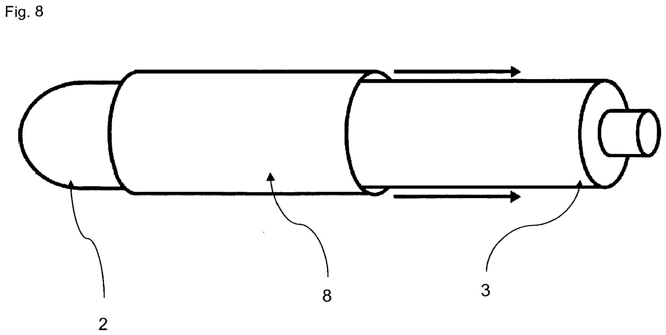

Fig. 8 stellt eine Seitenansicht eines Biopsie-Instrumentes mit einer Abdeckvorrichtung über einem Magnetischen Element dar. Hierbei ist die Abdeckvorrichtung 8 auf das Führungselement/Stabilisierungselement 6 aufgebracht und dient zur Abdeckung des Magnetischen Element es 4. Nach Anreicherung der Proben, wird die Abdeckvorrichtung in distaler Richtung über das Magnetischen Element 4 geschoben, wodurch das Magnetischen Element 4 und das angereicherte Probenmaterial bei der Ausfuhr des Biopsie-Instrumentes aus den Körperregionen geschützt und eine Kontamination vermieden wird. Es kann demgemäß eine hohe Qualität und Ausbeute erreicht werden.

### Bezugszeichenliste:

- 1: Führungselement
- 2: Distaler Bereich
- 3: Proximaler Bereich
- 4: Magnetisches Element
- 5: Stabilisierungselement
- 6: Führungselement/Stabilisierungselement
- 7: Aufnahmevorrichtung
- 8: Abdeckvorrichtung

## Patentansprüche

1. Ein Biopsie-Instrument zur Anreicherung von Probenmaterial, umfassend folgende Komponenten,
i) ein Führungselement (1), umfassend einen feder-elastischen distalen Bereich (2) und einen proximalen Bereich (3), und
ii) ein magnetisches Element (4),
**dadurch gekennzeichnet, dass**
das magnetische Element zwischen dem distalen Bereich (2) und dem proximalen Bereich (3) des Führungselementes (1) angebracht ist, und das Biopsie-Instrument
iii) ein mit dem proximalen Bereich (3) des Führungselementes (1) verbundenes Stabilisierungselement (5) umfasst.

2. Biopsie-Instrument nach Anspruch 1,
**dadurch gekennzeichnet, dass**
die Komponenten von distal nach proximal sequentiell angeordnet sind.

3. Biopsie-Instrument nach einem oder mehreren der vorhergehenden Ansprüche,
**dadurch gekennzeichnet, dass**
der distale Bereich (2) aus metallischen oder nicht-metallischen Materialien gefertigt ist.

4. Biopsie-Instrument nach einem oder mehreren der vorhergehenden Ansprüche,
**dadurch gekennzeichnet, dass**
das Stabilisierungselement (5) zylindrisch ausgebildet ist und/oder über den proximalen Teil des Führungselementes (1) schiebbar ist.

5. Biopsie-Instrument nach einem oder mehreren der vorhergehenden Ansprüche,
**dadurch gekennzeichnet, dass**
der Außendurchmesser des Stabilisierungselementes (5) gleich dem Außendurchmesser des distalen Bereichs (2) des Führungselementes (1) ist.

6. Biopsie-Instrument nach einem oder mehreren der vorhergehenden Ansprüche,
**dadurch gekennzeichnet, dass**
das magnetische Element (4) einen Außendurchmesser hat, der größer, gleich oder kleiner, vorzugsweise 0,01 bis 0,1 mm kleiner als der Außendurchmesser des distalen Bereichs (2) des Führungselementes (1) ist.

7. Biopsie-Instrument nach einem oder mehreren der vorhergehenden Ansprüche,
**dadurch gekennzeichnet, dass**
das magnetische Element (4) zusätzlich mit Detektionsmolekülen ausgestattet ist.

8. Biopsie-Instrument nach einem oder mehreren der vorhergehenden Ansprüche,
**dadurch gekennzeichnet, dass**
das Führungselement (1) zwischen dem distalen Bereich (2) und proximalen Bereich (3) eine Aufnahmevorrichtung (7) aufweist, welche der Aufnahme des magnetischen Elementes (4) dient.

9. Biopsie-Instrument nach einem oder mehreren der vorhergehenden Ansprüche,
**dadurch gekennzeichnet, dass**
das magnetisches Element (4) magnetische Werkstoffe enthält und dabei Metall, Kunststoff, und/oder keramischen Werkstoffen umfassen kann oder ein Elektromagneten ist.

10. Biopsie-Instrument nach Anspruch 7,
**dadurch gekennzeichnet, dass**
die Detektionsmoleküle ausgewählt aus der Gruppe umfassend Antikörper, Antikörperfragmente, Protein-Scaffolds, Peptide, Nukleinsäuren, Rezeptoren und/oder anorganischen Materialien sind.

11. Biopsie-Instrument nach einem oder mehreren der vorhergehenden Ansprüche,
**dadurch gekennzeichnet, dass**
der distale Bereich (2) des feder-elastischen Führungselementes (1) Detektionsmoleküle aufweist.

12. Biopsie-Instrument nach einem oder mehreren der vorhergehenden Ansprüche,
**dadurch gekennzeichnet, dass**
zusätzlich eine Abdeckvorrichtung (8) aufweist und/oder die Abdeckvorrichtung (8) vorzugsweise das magnetische Element (4) abdeckt, wobei die Abdeckvorrichtung (8) Homopolymere, Copolymere, Biopolymere, chemisch modifizierte Polymere und/oder synthetische Polymere umfasst.

13. Biopsie-Instrument nach einem oder mehreren der vorhergehenden Ansprüche,
**dadurch gekennzeichnet, dass**
es eine longitudinale Bohrung aufweist, zur Aufnahme bevorzugt eines Mandrains und/oder anderer Funktionselemente.

## Claims

1. A biopsy instrument for the enrichment of sample material comprising the following components:
i) a guide element (1) comprising a spring-elastic distal region (2) and a proximal region (3), and
ii) a magnetic element (4),
**characterized in that**
the magnetic element is positioned between the distal region (2) and the proximal region (3) of the guide element (1), and the biopsy instrument
(iii) comprises a stabilizing element (5) connected to the proximal region (3) of the guide element (1).

2. Biopsy instrument according to claim 1,
**characterized in that**
the components are sequentially arranged from distal to proximal.

3. Biopsy instrument according to one or more of the preceding claims,
**characterized in that**
the distal region (2) is made of metallic or non-metallic materials.

4. Biopsy instrument according to one or more of the preceding claims,
**characterized in that**
the stabilizing element (5) is cylindrically constructed and/or is extendable over the proximal part of the guide element (1).

5. Biopsy instrument according to one or more of the preceding claims,
**characterized in that**,
the outer diameter of the stabilizing element (5) is equal to the outer diameter of the distal region (2) of the guide element (1).

6. Biopsy instrument according to one or more of the preceding claims,
**characterized in that**
the magnetic element (4) has an outer diameter which is greater, equal to or smaller than, preferably 0.01 to 0.1 mm smaller than, the outer diameter of the distal region (2) of the guide element (1).

7. Biopsy instrument according to one or more of the preceding claims,
**characterized in that**
the magnetic element (4) is additionally equipped with detection molecules.

8. Biopsy instrument according to one or more of the preceding claims,
**characterized in that**
the guide element (1) has a receiving device (7) positioned between the distal region (2) and the proximal region (3), which serves for receiving the magnetic element (4).

9. Biopsy instrument according to one or more of the preceding claims,
**characterized in that**
the magnetic element (4) contains magnetic materials and can comprise metal, plastic, and/or ceramic materials or is an electromagnet.

10. Biopsy instrument according to claim 7,
**characterized in that**
the detection molecules are selected from the group comprising antibodies, antibody fragments, Protein-scaffolds, peptides, nucleic acids, receptors and/or inorganic materials.

11. Biopsy instrument according to one or more of the preceding claims,
**characterized in that**
the distal region (2) of the spring-elastic guide element (1) exhibits detection molecules.

12. Biopsy instrument according to one or more of the preceding claims,
**characterized in that**
the instrument additionally comprises a covering device (8) and/or the covering device (8) preferably covers the magnetic element (4), wherein the covering device (8) comprises homopolymers, copolymers, biopolymers, chemically modified polymers and/or synthetic polymers.

13. Biopsy instrument according to one or more of the preceding claims,
**characterized in that**
it has a longitudinal bore for receiving preferably a mandraine and/or other functional elements.

## Revendications

1. Instrument de biopsie servant à administrer un échantillon, comprenant des composants suivants,
i) un élément de guidage (1) comprenant une zone distale élastique à ressorts (2) et une zone proximale (3), et
ii) un élément magnétique (4),
**caractérisé en ce**
**que** l'élément magnétique est installé entre la zone distale (2) et la zone proximale (3) de l'élément de guidage (1), et en ce que l'instrument de biopsie comprend
iii) un élément de stabilisation (5) relié à la zone proximale (3) de l'élément de guidage (1).

2. Instrument de biopsie selon la revendication 1,
**caractérisé en ce**
**que** les composants sont disposés en série en partant du côté distal vers le côté proximal.

3. Instrument de biopsie selon l'une quelconque ou plusieurs des revendications précédentes,
**caractérisé en ce**
**que** la zone distale (2) est produite à partir de matières métalliques ou non métalliques.

4. Instrument de biopsie selon l'une quelconque ou plusieurs des revendications précédentes,
**caractérisé en ce**
**que** l'élément de stabilisation (5) est réalisé de manière cylindrique et/ou peut être glissé sur la partie proximale de l'élément de guidage (1).

5. Instrument de biopsie selon l'une quelconque ou plusieurs des revendications précédentes,
**caractérisé en ce**
**que** le diamètre extérieur de l'élément de stabilisation (5) est égal au diamètre extérieur de la zone distale (2) de l'élément de guidage (1).

6. Instrument de biopsie selon l'une quelconque ou plusieurs des revendications précédentes,
**caractérisé en ce**
**que** l'élément (4) magnétique a un diamètre extérieur, qui est plus grand, égal ou plus petit, de préférence inférieur de 0,01 à 0,1 mm, par rapport au diamètre extérieur de la zone distale (2) de l'élément de guidage (1).

7. Instrument de biopsie selon l'une quelconque ou plusieurs des revendications précédentes,
**caractérisé en ce**
**que** l'élément magnétique (4) est équipé en supplément de molécules de détection.

8. Instrument de biopsie selon l'une quelconque ou plusieurs des revendications précédentes,
**caractérisé en ce**
**que** l'élément de guidage (1) présente, entre la zone distale (2) et la zone proximale (3), un dispositif de réception (7), qui sert à recevoir l'élément magnétique (4).

9. Instrument de biopsie selon l'une quelconque ou plusieurs des revendications précédentes,
**caractérisé en ce**
**que** l'élément magnétique (4) contient des matériaux magnétiques et peut ce faisant comprendre du métal, une matière plastique et/ou des matériaux céramiques ou est un électroaimant.

10. Instrument de biopsie selon la revendication 7,
**caractérisé en ce**
**que** les molécules de détection sont choisies parmi le groupe comprenant les anticorps, les fragments d'anticorps, les protéines d'échafaudage, les peptides, les acides nucléiques, les récepteurs et/ou des matières inorganiques.

11. Instrument de biopsie selon l'une quelconque ou plusieurs des revendications précédentes,
**caractérisé en ce**
**que** la zone distale (2) de l'élément de guidage (1) élastique à ressorts présente des molécules de détection.

12. Instrument de biopsie selon l'une quelconque ou plusieurs des revendications précédentes,
**caractérisé en ce**
**qu'**il présente en supplément un dispositif de recouvrement (8), et/ou en ce que le dispositif de recouvrement (8) recouvre de préférence l'élément magnétique (4), dans lequel le dispositif de recouvrement (8) comprend des homopolymères, des copolymères, des biopolymères, des polymères modifiés chimiquement et/ou des polymères de synthèse.

13. Instrument de biopsie selon l'une quelconque ou plusieurs des revendications précédentes,
**caractérisé en ce**
**qu'**il présente un alésage longitudinal servant à recevoir de manière préférée un mandrin et/ou d'autres éléments fonctionnels.
